# EUROPÄISCHE PATENTSCHRIFT

(11) **EP 3 284 512 B1**
(45) Veröffentlichungstag und Bekanntmachung des Hinweises auf die Patenterteilung: **13.11.2019**
(21) Anmeldenummer: 16184554.0
(22) Anmeldetag: 17.08.2016
(51) Int. Cl.: A61N 1/375

(54) **ELEKTRISCHE DURCHFÜHRUNG MIT EINER GESINTERTEN ELEKTRISCHEN VERBINDUNG, INSBESONDERE FÜR MEDIZINISCHE IMPLANTATE**
ELECTRIC FEEDTHROUGH WITH A SINTERED ELECTRICAL CONNECTION, PARTICULARLY FOR MEDICAL IMPLANTS
PASSAGE ELECTRIQUE COMPRENANT UNE LIAISON ELECTRIQUE FRITTEE EN PARTICULIER POUR IMPLANTS MEDICAUX

(43) Veröffentlichungstag der Anmeldung: 21.02.2018
(73) Patentinhaber: Heraeus Deutschland GmbH & Co. KG, 63450 Hanau (DE)
(72) Erfinder: Hausch, Ulrisch, 60318 Frankfurt (DE); Schäfer, Michael, 36093 Künzell (DE); Nachreiner, Jens, 36381 Schlüchtern (DE)
(74) Vertreter: Herzog IP Patentanwalts GmbH

(56) Entgegenhaltungen:
- US-A1- 2012 193 141
- US-A1- 2012 194 981
- US-A1- 2013 338 750
- US-A1- 2015 122 875

## Beschreibung

Die vorliegende Erfindung betrifft eine Vorrichtung, beinhaltend einen ersten Rahmen, einen weiteren Rahmen, ein erstes Bauteil, ein zweites Bauteil und ein drittes Bauteil, wobei das dritte Bauteil eine elektrisch leitende Verbindung zwischen dem ersten Bauteil und dem zweiten Bauteil herstellt und eine Porosität in einem Bereich von 0,001 bis 0,4 hat; einen Vorrichtungsvorläufer, beinhaltend einen ersten Rahmen, einen weiteren Rahmen und ein erstes Bauteil, wobei eine Kontaktoberfläche des ersten Bauteils mindestens teilweise mit einer Zusammensetzung überlagert ist; ein Verfahren, beinhaltend ein Bereitstellen einer Durchführung, ein Erhalten eines Bauteilvorläufers und ein Erwärmen des Bauteilvorläufers; eine Vorrichtung, erhältlich durch das vorgenannte Verfahren; ein implantierbares elektrisches medizinisches Gerät; ein Verfahren zum Herstellen eines implantierbaren elektrischen medizinischen Geräts; ein implantierbares elektrisches medizinisches Gerät, erhältlich durch das vorstehende Verfahren; und eine Verwendung einer Zusammensatzung zu einem elektrisch leitenden Verbinden eines Durchführungselements und eines elektrischen Leiters.

Um eine elektrische Verbindung zwischen einem Innenraum eines hermetisch dichten Gehäuses eines elektrischen Geräts und einem Außenraum dieses Gehäuses zu schaffen, werden im Stand der Technik üblicherweise elektrische Durchführungen (feedthroughs) eingesetzt. Elektrische Geräte mit einem hermetisch dichten Gehäuse sind insbesondere elektrische medizinische implantierbare Geräte. Diese können aktive, elektrische Impulse gebende Geräte sein wie zum Beispiel Schrittmacher, Defibrillatoren oder Cochleaimplantate; aber auch passive, elektrische Signale messende Gerät wie EKG-Geräte. Im Stand der Technik bekannte Schrittmacher sind beispielsweise Blasenschrittmacher, Zwerchfellschrittmacher, Darmschrittmacher, Atemschrittmacher, Hirnschrittmacher und Herzschrittmacher. Bei den vorgenannten implantierbaren medizinischen Geräten ist das hermetisch dichte Gehäuse typischerweise ein Metallgehäuse, welches auf einer dem Innenraum oder dem Außenraum zugewandten Seite einen Header oder Headerblock, also einen Anschlusskörper mit Anschlussbuchsen zum Anschließen von elektrischen Leitungen, aufweist. Bei einem Herzschrittmacher weisen die Anschlussbuchsen elektrische Kontakte auf, über die Elektrodenleitungen im Außenraum elektrisch mit einer Steuerelektronik im Innenraum des hermetisch dichten Gehäuses verbunden werden können. Für implantierbare Geräte ist die hermetische Dichtigkeit von größter Bedeutung. Somit muss auch jede elektrische Durchführung hermetisch dicht sein. Eine solche Durchführung beinhaltet üblicherweise eine den Innenraum und den Außenraum elektrisch verbindende elektrische Leitung, welche durch einen elektrisch leitenden Anschlussstift gebildet wird, der durch eine Durchgangsöffnung in einem elektrisch isolierenden Isolationskörper hindurchtritt. Der elektrisch leitende Anschlussstift steht auf beiden Seiten über die jeweilige Stirnfläche des Isolationskörpers über, so dass auf beiden Seiten des Isolationskörpers und damit auf beiden Seiten der elektrischen Durchführung jeweils weiterführende elektrische Leitungen an den Anschlussstift angeschlossen werden können. Dieses Anschließen wird im Stand der Technik wie in EP 1 897 589 A2 und in US 2015/0122875 A1 vorgeschlagen durch Löten oder Schweißen realisiert.

Die elektrischen Durchführungen des Stands der Technik beinhalten mindestens folgende Nachteile. Eine elektrische Leitfähigkeit einer an einen Anschlussstift gelöteten oder geschweißten elektrischen Verbindung ist begrenzt, zum Beispiel durch eine Auswahl geeigneter Lote oder durch eine Mindestdicke der Löt- oder Schweißverbindung. Ferner weisen gelötete oder geschweißte elektrische Verbindungen regelmäßig einen Meniskus auf, der zu einer elektrischen Fehlfunktion, insbesondere zu einem Kurzschluss, der Durchführung führen kann. Demnach begrenzt ein solcher Meniskus eine Miniaturisierung von elektrischen Durchführungen. Eine gelötete oder geschweißte elektrische Verbindung an einer Durchführung muss nach dem Löten bzw. Schweißen gereinigt werden. Dies birgt ein Risiko dafür Verunreinigungen einzubringen. Zum Anschließen von elektrischen Leitungen an Durchführungen des Stands der Technik müssen Metalllegierungen oder Metallmischungen verwendet werden. Dies kann zum Bilden intermetallischer Phasen und damit zu Rissen oder zu einer verminderten Haltbarkeit der Verbindung und damit der Durchführung führen. Alle genannten Risiken, Defekte oder Haltbarkeitsbeschränkungen, die sich aus den elektrischen Durchführungen des Stands der Technik ergeben, sind besonders bedeutsam für implantierbare medizinische Geräte, da hier das Wohl des Patienten durch Fehlfunktionen oder die Notwendigkeit eines Austauschens eines defekten Gerätes direkt betroffen ist. Ferner ist ein elektrisches Verbinden eines Cermet-Anschlussstiftes einer Durchführung im Stand der Technik nicht oder nur unter großem Aufwand möglich. Ein Löten von porösen Materialien wie dem Cermet birgt die Gefahr, dass das eingesetzte Lot von den Poren des Materials aufgesogen wird.

Allgemein ist es eine Aufgabe der vorliegenden Erfindung, einen Nachteil, der sich aus dem Stand der Technik ergibt, zumindest teilweise zu überwinden. Eine weitere Aufgabe der Erfindung ist es, ein implantierbares elektrisches medizinisches Gerät mit einer verbesserten elektrischen Verbindung zwischen einer Cermet-Durchführung und einer elektrischen Leitung bereitzustellen. Eine weitere Aufgabe der Erfindung ist es eine Durchführung für ein implantierbares elektrisches medizinisches Gerät bereitzustellen, welche einen geringeren elektrischen Widerstand aufweist. Eine weitere Aufgabe der Erfindung ist es, ein implantierbares elektrisches medizinisches Gerät mit einer elektrischen Verbindung zu einer elektrischen Durchführung bereitzustellen, wobei die elektrische Verbindung keinen Meniskus beinhaltet. Eine weitere Aufgabe der Erfindung ist es ein implantierbares elektrisches medizinisches Gerät mit einem geringeren Risiko für einen Defekt, wie einen Kurzschluss, bereitzustellen. Eine weitere Aufgabe der Erfindung ist es ein implantierbares elektrisches medizinisches Gerät mit einem geringeren Pitch einer Durchführung bereitzustellen. Eine weitere Aufgabe der Erfindung ist es ein implantierbares elektrisches medizinisches Gerät mit einer Durchführung bereitzustellen, wobei ein Abstand zwischen einem Anschlussstift der Durchführung zu einer daran angeschlossen elektrischen Leitung geringer ist. Eine weitere Aufgabe der Erfindung ist es eine miniaturisierte elektrische Durchführung für ein implantierbares elektrisches medizinisches Gerät bereitzustellen. Eine weitere Aufgabe der Erfindung ist es, ein implantierbares elektrisches medizinisches Gerät mit einer elektrischen Verbindung zu einer elektrischen Durchführung bereitzustellen, wobei die elektrische Verbindung aus einem reinen Metall besteht oder keine Metalllegierung beinhaltet oder beides. Eine weitere Aufgabe der Erfindung ist es, ein implantierbares elektrisches medizinisches Gerät mit einer elektrischen Verbindung zu einer elektrischen Durchführung bereitzustellen, wobei die elektrische Verbindung durch eine höhere Haltbarkeit oder ein geringeres Risiko für Materialdefekte, wie Risse, oder beides charakterisiert ist. Eine Aufgabe der Erfindung ist es, ein implantierbares elektrisches medizinisches Gerät bereitzustellen, welches weniger aufwendig oder günstiger oder beides herzustellen ist. Eine weitere Aufgabe der Erfindung ist es, ein weniger aufwendiges oder günstigeres oder beides Herstellungsverfahren eines implantierbaren elektrischen medizinischen Geräts bereitzustellen. Eine weitere Aufgabe der Erfindung ist es, ein implantierbares elektrisches medizinisches Gerät mit einem erhöhten Wirkungsgrad bereitzustellen. Eine weitere Aufgabe der Erfindung ist es, ein implantierbares elektrisches medizinisches Gerät mit einem geringeren Energieverbrauch bereitzustellen. Eine weitere Aufgabe der Erfindung ist es, ein implantierbares elektrisches medizinisches Gerät mit einer längeren Batterielebensdauer bereitzustellen. Eine weitere Aufgabe der Erfindung ist es, ein implantierbares elektrisches medizinisches Gerät mit einer erhöhten Betriebssicherheit bereitzustellen. Eine weitere Aufgabe der Erfindung ist es, ein implantierbares elektrisches medizinisches Gerät mit einer verringerten Anzahl von Verbindungsstellen bereitzustellen. Eine weitere Aufgabe der Erfindung ist es, ein implantierbares elektrisches medizinisches Gerät mit einer erhöhten Funktionsdauer bereitzustellen. Eine weitere Aufgabe der Erfindung ist es, ein weniger störanfälliges implantierbares elektrisches medizinisches Gerät bereitzustellen. Eine weitere Aufgabe der Erfindung ist es, ein Herstellungsverfahren für eines der obigen vorteilhaften implantierbaren elektrischen medizinischen Geräte bereitzustellen. Eine weitere Aufgabe der Erfindung ist es, ein verbessertes implantierbares elektrisches medizinisches Gerät bereitzustellen. Eine weitere Aufgabe der Erfindung ist es, eine verbesserte Therapie von Bradykardie oder Tachykardie oder beidem bereitzustellen. Eine weitere Aufgabe der Erfindung ist es, eine kostengünstigere Therapie von Bradykardie oder Tachykardie oder beidem bereitzustellen. Eine weitere Aufgabe der Erfindung ist es, eine schonendere Therapie von Bradykardie oder Tachykardie oder beidem bereitzustellen. Eine weitere Aufgabe der Erfindung ist es, eine Therapie für Bradykardie oder Tachykardie oder beidem, welche weniger chirurgische Eingriffe beinhaltet, bereitzustellen. Eine weitere Aufgabe der Erfindung ist es, eine risikoärmere Therapie von Bradykardie oder Tachykardie oder beidem bereitzustellen. Eine weitere Aufgabe der Erfindung ist es, ein Verfahren zu einem elektrischen Verbinden einer Durchführung eines implantierbaren elektrischen medizinischen Geräts mit einer elektrischen Leitung bereitzustellen, wobei das Verfahren ein geringeres Risiko für ein Einbringen von Verunreinigungen birgt. Eine weitere Aufgabe der Erfindung ist es, ein Verfahren zu einem verbesserten elektrischen Verbinden einer Cermet-Durchführung eines implantierbaren elektrischen medizinischen Geräts mit einer elektrischen Leitung bereitzustellen. Eine weitere Aufgabe der Erfindung ist es, einen Herzschrittmacher oder ein implantierbares EKG-Gerät oder beides, welcher oder welches einen der Vorteile oder eine ausgewogene Kombination zweier oder mehrerer der Vorteile der oben genannten implantierbaren elektrischen medizinischen Geräte aufweist, bereitzustellen.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen.

Einen Beitrag zur Erfüllung mindestens einer der erfindungsgemäßen Aufgaben leistet eine Ausführungsform 1 einer Vorrichtung 1, beinhaltend einen ersten Rahmen, einen weiteren Rahmen, ein erstes Bauteil, ein zweites Bauteil und ein drittes Bauteil;
wobei
a) der erste Rahmen den weiteren Rahmen umrahmt;
b) der weitere Rahmen
   i) das erste Bauteil umrahmt, und
   ii) das erste Bauteil und den ersten Rahmen elektrisch voneinander isoliert;
c) das erste Bauteil elektrisch leitend ist;
d) das zweite Bauteil elektrisch leitend ist; und
e) das dritte Bauteil
   i) eine elektrisch leitende Verbindung zwischen dem ersten Bauteil und dem zweiten Bauteil herstellt, und
   ii) eine Porosität in einem Bereich von 0,001 bis 0,4, bevorzugt von 0,01 bis 0,3, bevorzugter von 0,05 bis 0,25, bevorzugter von 0,1 bis 0,2, am bevorzugtesten von 0,13 bis 0,16, hat.

Eine erfindungsgemäße Ausführungsform 2 der Vorrichtung 1 ist nach der Ausführungsform 1 ausgestaltet, wobei das erste Bauteil ein Cermet beinhaltet, bevorzugt daraus besteht.

Eine erfindungsgemäße Ausführungsform 3 der Vorrichtung 1 ist nach der Ausführungsform 1 oder 2 ausgestaltet, wobei der weitere Rahmen eine Keramik beinhaltet, bevorzugt daraus besteht.

Eine erfindungsgemäße Ausführungsform 4 der Vorrichtung 1 ist nach einer der vorhergehenden Ausführungsformen ausgestaltet, wobei der weitere Rahmen und das erste Bauteil einstückig ausgebildet sind. Bevorzugt bestehen der weitere Rahmen und das erste Bauteil aus voneinander verschiedenen Materialien, welche materialschlüssig miteinander verbunden sind. Eine bevorzugte materialschlüssige Verbindung beinhaltet keine Lötstellen oder keine Schweißstellen oder beides. Bevorzugt wurden der weitere Rahmen und das erste Bauteil in einem Herstellungsverfahren als ein Stück hergestellt, ohne ein Zusammenfügen vorgefertigter Teile.

Eine erfindungsgemäße Ausführungsform 5 der Vorrichtung 1 ist nach einer der vorhergehenden Ausführungsformen ausgestaltet, wobei das zweite Bauteil eines ausgewählt aus der Gruppe bestehend aus einem elektrischen Kontakt, einer Leiterbahn, einem Draht, einer Buchse, und einem Stecker, oder eine Kombination von mindestens zwei davon ist.

Eine erfindungsgemäße Ausführungsform 6 der Vorrichtung 1 ist nach einer der vorhergehenden Ausführungsformen ausgestaltet, wobei das erste Bauteil oder das zweite Bauteil oder beide mit einem elektrotechnischen Filter verbunden ist. Bevorzugt ist das erste Bauteil oder das zweite Bauteil oder beide mechanisch oder elektrisch leitend oder beides mit dem elektrotechnischen Filter verbunden. Ein bevorzugter elektrotechnischer Filter ist ein MLCC-Filter. Bevorzugt ist der elektrotechnische Filter auf einer Seites des ersten Rahmes, die dem zweiten Bauteil zugewandt ist, angeordnet.

Eine erfindungsgemäße Ausführungsform 7 der Vorrichtung 1 ist nach einer der vorhergehenden Ausführungsformen ausgestaltet, wobei das dritte Bauteil eines oder mindestens zwei, bevorzugt mindestens drei, bevorzugter mindestens 4, bevorzugter mindestens 5, bevorzugter mindestens 6, am bevorzugtesten mindestens 7, der folgenden Kriterien erfüllt:
a) ein elektrischer Widerstand von maximal 0,02 mΩ·cm, bevorzugt von maximal 0,015 mΩ·cm, am bevorzugtesten von maximal 0,01 mΩ·cm;
b) eine Schichtdicke in einem Bereich von 0,005 bis 1 mm, bevorzugt von 0,01 bis 1 mm, bevorzugter von 0,05 bis 1 mm, bevorzugter von 0,1 bis 0,8 mm, am bevorzugtesten von 0,2 bis 0,6 mm;
c) besteht aus einem Metall oder einem Metallgemisch oder beidem, wobei das dritte Bauteil eine Rohdichte in einem Bereich von 60 bis 99,9 %, bevorzugt von 70 bis 99 %, bevorzugter von 75 bis 95 %, bevorzugter von 80 bis 90 %,am bevorzugtesten von 84 bis 87 %, bezogen auf die Reindichte des Metalls bzw. des Metallgemischs, hat;
d) beinhaltet nur ein Metall zu einem Anteil von mehr als 0,05 Gew.-%, bevorzugt von mehr als 0,03 Gew.-%, bevorzugter von mehr als 0,01 Gew.-%, jeweils bezogen auf das Gewicht des dritten Bauteils;
e) beinhaltet keinen Meniskus;
f) beinhaltet eines ausgewählt aus der Gruppe bestehend aus Ag, Au, Cu, Pt und Pd, oder eine Kombination von mindestens zwei davon zu einem Anteil in einem Bereich von 10 bis 100 Gew.-%, bevorzugt von 20 bis 100 Gew.-%, bevorzugter von 30 bis 100 Gew.-%, bevorzugter von 40 bis 100 Gew.-%, bevorzugter von 50 bis 100 Gew.-%, bevorzugter von 60 bis 100 Gew.-%, bevorzugter von 70 bis 100 Gew.-%, bevorzugter von 80 bis 100 Gew.-%, bevorzugter von 90 bis 100 Gew.-%, bevorzugter von 95 bis 100 Gew.-%, bevorzugter von 98 bis 100 Gew.-%, am bevorzugtesten von 99 bis 100 Gew.-%, bezogen auf das Gewicht des dritten Bauteils;
g) mindestens 50 %, bevorzugt mindestens 60 %, bevorzugter mindestens 70 %, bevorzugter mindestens 80 %, bevorzugter mindestens 90 %, bevorzugter mindestens 95 %, am bevorzugtesten 100 %, der Körner des dritten Bauteils haben eine Korngröße in einem Bereich von 1 bis 500 nm, bevorzugt von 5 bis 450 nm, bevorzugter von 10 bis 400 nm, bevorzugter von 50 bis 350 nm, bevorzugter von 100 bis 300 nm, am bevorzugtesten von 150 bis 250 nm;
h) mindestens 50 %, bevorzugt mindestens 60 %, bevorzugter mindestens 70 %, bevorzugter mindestens 80 %, bevorzugter mindestens 90 %, bevorzugter mindestens 95 %, am bevorzugtesten 100 %, der Poren des dritten Bauteils haben eine Porengröße in einem Bereich von 0,5 bis 1,5 µm, bevorzugt von 0,6 bis 0,9 µm, am bevorzugtesten von 0,6 bis 0,8 µm.

Bevorzugt erfüllt das dritte Bauteil jedes der obigen Kriterien. Unter dem obigen Punkt f) ist Ag oder Au oder beides besonders bevorzugt. Ist die erfindungsgemäße Vorrichtung eine elektrische Durchführung in einem Gehäuse,
a. so beinhaltet das dritte Bauteil bevorzugt Ag, wenn das zweite Bauteil sich in einem Inneren des Gehäuses befindet; und
b. so beinhaltet das dritte Bauteil bevorzugt Au, wenn das zweite Bauteil sich außerhalb des Gehäuses befindet.

Eine erfindungsgemäße Ausführungsform 8 der Vorrichtung 1 ist nach einer der vorhergehenden Ausführungsformen ausgestaltet, wobei die elektrisch leitende Verbindung einen elektrischen Widerstand in einem Bereich von 0,001 bis 10 mΩ, bevorzugt von 0,01 bis 9 mΩ, bevorzugter von 0,05 bis 8 mΩ, bevorzuget von 0,075 bis 7,5 mΩ, bevorzugter von 0,1 bis 6 mΩ, am bevorzugtesten von 0,5 bis 5 mΩ, hat.

Einen Beitrag zur Erfüllung mindestens einer der erfindungsgemäßen Aufgaben leistet eine Ausführungsform 1 eines Vorrichtungsvorläufers 1, beinhaltend einen ersten Rahmen, einen weiteren Rahmen und ein erstes Bauteil;
wobei
a) der erste Rahmen den weiteren Rahmen umrahmt;
b) der weitere Rahmen
   i) das erste Bauteil umrahmt, und
   ii) das erste Bauteil und den ersten Rahmen elektrisch voneinander isoliert; und
c) das erste Bauteil
   i) elektrisch leitend ist, und
   ii) eine erste Kontaktoberfläche beinhaltet;
   wobei die erste Kontaktoberfläche mindestens teilweise mit einer Zusammensetzung, beinhaltend eine Flüssigkeit und eine Vielzahl von Metallpartikeln, überlagert ist. Ein bevorzugter erster Rahmen ist ein erster Rahmen nach der erfindungsgemäßen Vorrichtung 1. Ein bevorzugter weiterer Rahmen ist ein weiterer Rahmen nach der erfindungsgemäßen Vorrichtung 1. Ein bevorzugtes erstes Bauteil ist ein erstes Bauteil nach der erfindungsgemäßen Vorrichtung 1.

Eine erfindungsgemäße Ausführungsform 2 des Vorrichtungsvorläufers 1 ist nach der Ausführungsform 1 ausgestaltet, wobei die Zusammensetzung eines oder mindestens zwei, bevorzugt mindestens drei, bevorzugter mindestens 4, bevorzugter mindestens 5, bevorzugter mindestens 6, am bevorzugtesten mindestens 7, der folgenden Kriterien erfüllt:
a) beinhaltet die Vielzahl von Metallpartikeln zu einem Anteil in einem Bereich von 75 bis 99 Gew.-%, bevorzugt 80 bis 98 Gew.-%, bevorzugter 85 bis 95 Gew.-%, am bevorzugtesten 88 bis 93 Gew.-%, jeweils bezogen auf das Gewicht der Zusammensetzung;
b) beinhaltet die Flüssigkeit zu einem Anteil in einem Bereich von 1 bis 25 Gew.-%, bevorzugt von 2 bis 20 Gew.-%, bevorzugter von 5 bis 15 Gew.-%, am bevorzugtesten von 7 bis 12 Gew.-%, jeweils bezogen auf das Gewicht der Zusammensetzung;
c) beinhaltet nur ein Metall zu einem Anteil von mehr als 0,05 Gew.-%, bevorzugt von mehr als 0,03 Gew.-%, bevorzugter von mehr als 0,01 Gew.-%, jeweils bezogen auf das Gewicht der Zusammensetzung;
d) hat eine Viskosität in einem Bereich von 50 bis 500 Pa·s, bevorzugt von 75 bis 450 Pa·s, bevorzugter von 100 bis 400 Pa·s, am bevorzugtesten von 150 bis 350 Pa·s;
e) mindestens ein Teil der Vielzahl der Metallpartikel beinhaltet eines ausgewählt aus der Gruppe bestehend aus Ag, Au, Cu, Pt, und Pd, oder eine Kombination von mindestens zwei davon zu einem Anteil in einem Bereich von 10 bis 100 Gew.-%, bevorzugt von 20 bis 100 Gew.-%, bevorzugter von 30 bis 100 Gew.-%, bevorzugter von 40 bis 100 Gew.-%, bevorzugter von 50 bis 100 Gew.-%, bevorzugter von 60 bis 100 Gew.-%, bevorzugter von 70 bis 100 Gew.-%, bevorzugter von 80 bis 100 Gew.-%, bevorzugter von 90 bis 100 Gew.-%, bevorzugter von 95 bis 100 Gew.-%, bevorzugter von 98 bis 100 Gew.-%, am bevorzugtesten von 99 bis 100 Gew.-%, bezogen auf das Gesamtgewicht des Teils der Vielzahl der Metallpartikel;
f) die Metallpartikel haben eine Partikelgrößenverteilung gekennzeichnet durch ein D₅₀ in einem Bereich von 0,1 bis 20µm, bevorzugt von 1 bis 15 µm, am bevorzugtesten von 2 bis 10 µm;
g) die Metallpartikel haben eine massenspezifische Oberfläche in einem Bereich von 0,5 bis 5 m²/kg, bevorzugt von 1 bis 4 m²/kg, am bevorzugtesten von 2 bis 3 m²/kg;
h) beinhaltet kein Halogen zu mehr als 1500 ppm, bevorzugt zu mehr als 1000 ppm, am bevorzugtesten zu mehr als 500 ppm, jeweils bezogen auf das Gewicht der Zusammensetzung.

Bevorzugt erfüllt die Zusammensetzung jedes der obigen Kriterien.

Einen Beitrag zur Erfüllung mindestens einer der erfindungsgemäßen Aufgaben leistet eine Ausführungsform 1 eines Verfahrens 1, beinhaltend als Verfahrensschritte:
a) Bereitstellen einer Durchführung, beinhaltend einen ersten Rahmen, einen weiteren Rahmen, und ein erstes Bauteil,
   wobei
   i) der erste Rahmen den weiteren Rahmen umrahmt,
   ii) der weitere Rahmen
      A. das erste Bauteil umrahmt, und
      B. den ersten Rahmen und das erste Bauteil elektrisch voneinander isoliert, und
   iii) das erste Bauteil
      A. elektrisch leitend ist, und
      B. eine erste Kontaktoberfläche beinhaltet;
b) Bereitstellen eines zweiten Bauteils, beinhaltend eine weitere Kontaktoberfläche;
c) Bereitstellen einer Zusammensetzung, beinhaltend eine Flüssigkeit und eine Vielzahl von Metallpartikeln,
   wobei die Metallpartikel ein Metall mit einer niedrigsten Schmelztemperatur beinhalten;
d) Überlagern der ersten Kontaktoberfläche oder der weiteren Kontaktoberfläche oder beider mit der Zusammensetzung;
e) Verbinden der ersten Kontaktoberfläche und der weiteren Kontaktoberfläche über die Zusammensetzung;
f) Verringern des Anteils der Flüssigkeit an der Zusammensetzung, bevorzugt um mindestens 80 Gew.-%, bevorzugter um mindestens 85 Gew.-%, bevorzugter um mindestens 90 Gew.-%, am bevorzugtesten um mindestens 95 Gew.-%, bezogen auf den Anteil der Flüssigkeit an der Zusammensetzung unmittelbar vor Verfahrensschritt f), unter Erhalt eines Bauteilvorläufers; und
g) Erwärmen des Bauteilvorläufers auf eine Temperatur in einem Bereich von 10 bis 90 %, bevorzugt von 10 bis 80 %, bevorzugter von 10 bis 70 %, bevorzugter von 10 bis 60 %, bevorzugter von 15 bis 50 %, bevorzugter von 15 bis 40 %, am bevorzugtesten von 15 bis 35 %, der niedrigsten Schmelztemperatur unter Erhalt eines dritten Bauteils.

Ein bevorzugtes Verringern in Verfahrensschritt f) ist ein Austreiben. Ein bevorzugtes Austreiben ist ein Entbindern. Ein bevorzugtes Entbindern ist eines ausgewählt aus der Gruppe bestehend aus einem thermischen, einem katalytischen, und einem Lösungsmittel-Entbindern, oder eine Kombination aus mindestens zwei davon. Ein bevorzugter Bauteilvorläufer ist ein Grünling oder ein Braunling oder beides. Ein bevorzugter erster Rahmen ist ein erster Rahmen nach der erfindungsgemäßen Vorrichtung 1. Ein bevorzugter weiterer Rahmen ist ein weiterer Rahmen nach der erfindungsgemäßen Vorrichtung 1. Ein bevorzugtes erstes Bauteil ist ein erstes Bauteil nach der erfindungsgemäßen Vorrichtung 1. Ein bevorzugtes zweites Bauteil ist ein zweites Bauteil nach der erfindungsgemäßen Vorrichtung 1. Ein bevorzugtes drittes Bauteil ist ein drittes Bauteil nach der erfindungsgemäßen Vorrichtung 1. Eine bevorzugte Zusammensetzung ist die Zusammensetzung nach dem erfindungsgemäßen Vorrichtungsvorläufer 1. Bevorzugt wird in dem Verfahrensschritt d) ein erfindungsgemäßer Vorrichtungsvorläufer 1 erhalten. In einer bevorzugten Ausführungsform erfolgt das Erwärmen in Verfahrensschritt g) auf eine Maximaltemperatur in einem Bereich von 150 bis 350°C, bevorzugt von 180 bis 320°C, bevorzugter von 200 bis 300°C, am bevorzugtesten von 220 bis 290°C. Die vorgenannte Maximaltemperatur wird bevorzugt für 0,5 bis 20 Minuten, bevorzugter 0,5 bis 7 Minuten, am bevorzugtesten 0,5 bis 5 Minuten gehalten. Weiterhin bevorzugt wird Verfahrensschritt g) in einer Sinterpresse ausgeführt.

Eine erfindungsgemäße Ausführungsform 2 des Verfahrens 1 ist nach der Ausführungsform 1 ausgestaltet, wobei das Verringern in Verfahrensschritt f) ein Erwärmen der Zusammensetzung auf eine Temperatur in einem Bereich von 50 bis 160 °C, bevorzugt von 70 bis 150°C, bevorzugter von 80 bis 130°C, am bevorzugtesten von 90 bis 110°C, beinhaltet. Die vorgenannte Temperatur ist bevorzugt eine Maximaltemperatur in Verfahrensschritt f). Die vorgenannte Temperatur wird bevorzugt für 0,5 bis 30 Minuten, bevorzugter 1 bis 25 Minuten, am bevorzugtesten 3 bis 20 Minuten gehalten. In Verfahrensschritt f) befindet sich der Bauteilvorläufer bevorzugt in Luft. Weiterhin bevorzugt wird Verfahrensschritt f) in einem Kammerofen oder einem Kammertrockner oder in beiden ausgeführt.

Eine erfindungsgemäße Ausführungsform 3 des Verfahrens 1 ist nach der Ausführungsform 1 oder 2 ausgestaltet, wobei Verfahrensschritt g) eines oder mindestens 2, bevorzugt mindestens 3, der folgenden Kriterien erfüllt:
a) der Bauteilvorläufer wird in Verfahrensschritt g) einem mechanischen Druck in einem Bereich von 0 bis 50 MPa, bevorzugt von 5 bis 45 MPa, bevorzugter von 10 bis 40 MPa, am bevorzugtesten von 15 bis 35 MPa, ausgesetzt;
b) während das dritte Bauteil erhalten wird befindet sich der Bauteilvorläufer oder das dritte Bauteil oder beide in einer Schutzgasatmosphäre;
c) ein Abstand der ersten Kontaktoberfläche von der weiteren Kontaktoberfläche verringert sich in Verfahrensschritt g) um 0,01 bis 5 %, bevorzugt um 0,05 bis 4,5 %, bevorzugter um 0,1 bis 4 %, bevorzugter um 0,5 bis 3,5 %, am bevorzugtesten um 1 bis 3 %, bezogen auf den Abstand unmittelbar vor dem Verfahrensschritt g), auf einen Wert in einem Bereich von 0,005 bis 1 mm, bevorzugt von 0,01 bis 1 mm, bevorzugter von 0,05 bis 1 mm, bevorzugter von 0,1 bis 0,8 mm, am bevorzugtesten von 0,2 bis 0,6 mm;
d) die in Verfahrensschritt g) genannte Temperatur wird für eine Zeitdauer in einem Bereich von 0,5 bis 120 min, bevorzugt von 0,7 bis 50 min, bevorzugter von 1 bis 20 min, bevorzugter von 1 bis 10 min, bevorzugter von 1 bis 5 min gehalten.

Bevorzugt erfüllt der Verfahrensschritt g) eine der folgenden Kombinationen der obigen Kriterien: a)b)c)d), und a)c)d).

Ein bevorzugter mechanischer Druck wird von einem Festkörper, bevorzugt einer Druckgussform, auf den Bauteilvorläufer ausgeübt. Eine bevorzugte Schutzgasatmosphäre beinhaltet eines aus der Gruppe bestehend aus:
- Sauerstoff zu einem Anteil von weniger als 20 Gew.-%, bevorzugt weniger als 15 Gew.-%, bevorzugter weniger 10 Gew.-%, am bevorzugtesten weniger als 5 Gew.-%, bezogen auf die Schutzgasatmosphäre;
- H₂O zu einem Anteil von weniger als 5 Gew.-%, bevorzugt weniger als 4 Gew.-%, bevorzugter weniger als 3 Gew.-%, am bevorzugtesten weniger als 2 Gew.-%, bezogen auf die Schutzgasatmosphäre;
- CO zu einem Anteil von weniger als 1 Gew.-%, bevorzugt weniger als 0,8 Gew.-%, am bevorzugtesten weniger als 0,5 Gew.-%, bezogen auf die Schutzgasatmosphäre; und CO₂ zu einem Anteil von weniger als 4 Gew.-%, bevorzugt weniger als 3 Gew.-%, bevorzugter weniger als 2 Gew.-%, am bevorzugtesten weniger als 1 Gew.-%, bezogen auf die Schutzgasatmosphäre; oder
- eine Kombination aus mindestens zwei davon.

Eine weitere bevorzugte Schutzgasatmosphäre ist gekennzeichnet durch einen Gasdruck in einem Bereich von 0,1 bis 1100 mbar, bevorzugt von 0,5 bis 1000 mbar, bevorzugter von 1 bis 900 mbar, bevorzugter von 10 bis 800 mbar bevorzugter von 50 bis 700 mbar, bevorzugter von 100 bis 600 mbar, am bevorzugtesten von 200 bis 500 mbar. In einer weiteren bevorzugten Ausgestaltung befindet sich der Bauteilvorläufer in Verfahrensschritt g) in Luft.

Eine erfindungsgemäße Ausführungsform 4 des Verfahrens 1 ist nach einer der Ausführungsformen 1 bis 3 ausgestaltet, wobei das Überlagern eines ausgewählt aus der Gruppe bestehend aus einem Dispensieren, einem Dippen, einem Drucken, einem Streichen, und einem Rakeln oder eine Kombination von mindestens zwei davon ist. Ein bevorzugtes Drucken ist ein Schablonendrucken. Bei dem Schablonendrucken wird die Zusammensetzung durch mindestens eine Öffnung, bevorzugt durch eine Vielzahl von Öffnungen, in einer Schablone gedruckt.

Eine erfindungsgemäße Ausführungsform 5 des Verfahrens 1 ist nach einer der Ausführungsformen 1 bis 4 ausgestaltet, wobei nach Verfahrensschritt g) das dritte Bauteil nicht gereinigt wird. Dabei ist ein Reinigen ein Behandeln des dritten Bauteils mit dem Ziel eine Verunreinigung auf dem dritten Bauteil zu verringern oder zu entfernen oder beides. Bevorzugt wird das dritte Bauteil nach Verfahrensschritt g) nicht mit einem Reinigungsmittel oder einem Reinigungswerkzeug oder beidem kontaktiert. Ein bevorzugtes Reinigungsmittel ist eine Lösung, beinhaltend zu einem Anteil von mindestens 40 Gew.-%, bevorzugt mindestens 50 Gew.-%, bevorzugter mindestens 60 Gew.-%, bevorzugter mindestens 70 Gew.-%, bevorzugter mindestens 80 Gew.-%, am bevorzugtesten mindestens 90 Gew.-%, ein Lösungsmittel; oder ein Flussmittel, oder beides. Ein bevorzugtes Lösungsmittel ist ein Alkohol oder Aceton oder beides. Ein bevorzugter Alkohol ist Isopropanol. Ein bevorzugtes Reinigungswerkzeug ist ein Glasradierer.

Eine erfindungsgemäße Ausführungsform 6 des Verfahrens 1 ist nach einer der Ausführungsformen 1 bis 5 ausgestaltet, wobei die Zusammensetzung eines oder mindestens zwei, bevorzugt mindestens drei, bevorzugter mindestens 4, bevorzugter mindestens 5, bevorzugter mindestens 6, am bevorzugtesten mindestens 7, der folgenden Kriterien erfüllt:
a) beinhaltet die Vielzahl von Metallpartikeln zu einem Anteil in einem Bereich von 75 bis 99 Gew.-%, bevorzugt 80 bis 98 Gew.-%, bevorzugter 85 bis 95 Gew.-%, am bevorzugtesten 88 bis 93 Gew.-%, bezogen auf das Gewicht der Zusammensetzung;
b) beinhaltet die Flüssigkeit zu einem Anteil in einem Bereich von 1 bis 25 Gew.-%, bevorzugt von 2 bis 20 Gew.-%, bevorzugter von 5 bis 15 Gew.-%, am bevorzugtesten von 7 bis 12 Gew.-%, jeweils bezogen auf das Gewicht der Zusammensetzung;
c) beinhaltet nur ein Metall zu einem Anteil von mehr als 0,05 Gew.-%, bevorzugt von mehr als 0,03 Gew.-%, bevorzugter von mehr als 0,01 Gew.-%, jeweils bezogen auf das Gewicht der Zusammensetzung;
d) hat eine Viskosität in einem Bereich von 50 bis 500 Pa·s, bevorzugt von 75 bis 450 Pa·s, bevorzugter von 100 bis 400 Pa·s, am bevorzugtesten von 150 bis 350 Pa·s;
e) mindestens ein Teil der Vielzahl der Metallpartikel beinhaltet eines ausgewählt aus der Gruppe bestehend aus Ag, Au, Cu, Pt, und Pd, oder eine Kombination von mindestens zwei davon zu einem Anteil in einem Bereich von 10 bis 100 Gew.-%, bevorzugt von 20 bis 100 Gew.-%, bevorzugter von 30 bis 100 Gew.-%, bevorzugter von 40 bis 100 Gew.-%, bevorzugter von 50 bis 100 Gew.-%, bevorzugter von 60 bis 100 Gew.-%, bevorzugter von 70 bis 100 Gew.-%, bevorzugter von 80 bis 100 Gew.-%, bevorzugter von 90 bis 100 Gew.-%, bevorzugter von 95 bis 100 Gew.-%, bevorzugter von 98 bis 100 Gew.-%, am bevorzugtesten von 99 bis 100 Gew.-%, bezogen auf das Gesamtgewicht des Teils der Vielzahl der Metallpartikel;
f) die Metallpartikel haben eine Partikelgrößenverteilung gekennzeichnet durch ein D₅₀ in einem Bereich von 0,1 bis 20 µm, bevorzugt von 1 bis 15 µm, am bevorzugtesten von 2 bis 10 µm;
g) die Metallpartikel haben eine massenspezifische Oberfläche in einem Bereich von 0,5 bis 5 m²/kg, bevorzugt von 1 bis 4 m²/kg, am bevorzugtesten von 2 bis 3 m²/kg;
h) beinhaltet kein Halogen zu mehr als 1500 ppm, bevorzugt zu mehr als 1000 ppm, am bevorzugtesten zu mehr als 500 ppm, jeweils bezogen auf das Gewicht der Zusammensetzung.

Bevorzugt erfüllt die Zusammensetzung jedes der obigen Kriterien.

Einen Beitrag zur Erfüllung mindestens einer der erfindungsgemäßen Aufgaben leistet eine Ausführungsform 1 einer Vorrichtung 2, erhältlich durch das Verfahren 1 nach einer seiner Ausführungsformen 1 bis 5. Bevorzugte Komponenten und Bestandteile der Vorrichtung 2 sind ausgestaltet gemäß einer der Ausführungsformen der erfindungsgemäßen Vorrichtung 1.

Einen Beitrag zur Erfüllung mindestens einer der erfindungsgemäßen Aufgaben leistet eine Ausführungsform 1 eines implantierbaren elektrischen medizinischen Geräts 1, beinhaltend ein Gehäuse und eine Vorrichtung 1 nach einer ihrer Ausführungsformen 1 bis 8, oder eine Vorrichtung 2 nach ihrer Ausführungsform 1; wobei das Gehäuse eine Gehäuseöffnung beinhaltet; wobei die Gehäuseöffnung den ersten Rahmen beinhaltet. Bevorzugt ist das Gehäuse hermetisch dicht. Bevorzugt beinhaltet dichtet die Vorrichtung 1 oder die Vorrichtung 2 die Gehäuseöffnung hermetisch dicht.

Einen Beitrag zur Erfüllung mindestens einer der erfindungsgemäßen Aufgaben leistet eine Ausführungsform 1 eines Verfahrens 2 zum Herstellen eines implantierbaren elektrischen medizinischen Geräts, beinhaltend ein Gehäuse, beinhaltend eine Gehäuseöffnung, wobei in einem Verfahrensschritt eine Vorrichtung 1 nach einer ihrer Ausführungsformen 1 bis 8, oder eine Vorrichtung 2 nach ihrer Ausführungsform 1, oder ein Vorrichtungsvorläufer 1 nach einer seiner Ausführungsformen 1 oder 2 so in die Gehäuseöffnung eingebracht wird, dass die Gehäuseöffnung den ersten Rahmen beinhaltet. Bevorzugt wird die Vorrichtung 1, oder die Vorrichtung 2, oder der Vorrichtungsvorläufer 1 so in die Gehäuseöffnung eingebracht, dass die Gehäuseöffnung hermetisch dicht verschlossen wird. Dazu kann die Vorrichtung 1, oder die Vorrichtung 2, oder der Vorrichtungsvorläufer 1 in die Gehäuseöffnung eingelötet oder eingeschweißt oder beides werden. Bevorzugt besteht der erste Rahmen aus demselben Material wie das Gehäuse und wird in die Gehäuseöffnung eingeschweißt.

Einen Beitrag zur Erfüllung mindestens einer der erfindungsgemäßen Aufgaben leistet eine Ausführungsform 1 eines implantierbaren elektrischen medizinischen Geräts 2, erhältlich durch das Verfahren 2 nach seiner Ausführungsform 1.

Einen Beitrag zur Erfüllung mindestens einer der erfindungsgemäßen Aufgaben leistet eine Ausführungsform 1 einer Verwendung 1 einer Zusammensatzung, beinhaltend eine Flüssigkeit und eine Vielzahl von Metallpartikeln, zu einem elektrisch leitenden Verbinden eines Durchführungselements und eines elektrischen Leiters; wobei die Verwendung ein Sintern der Zusammensetzung beinhaltet. Eine bevorzugte Zusammensetzung ist die Zusammensetzung nach dem erfindungsgemäßen Vorrichtungsvorläufer 1 oder dem erfindungsgemäßen Verfahren 1 oder beidem.

Einen Beitrag zur Erfüllung mindestens einer der erfindungsgemäßen Aufgaben leistet eine Ausführungsform 1 eines Verfahrens 3, beinhaltend als Verfahrensschritte
a) Bereitstellen eines implantierbaren elektrischen medizinischen Geräts 1 nach seiner Ausführungsform 1 oder eines implantierbaren elektrischen medizinischen Geräts 2 nach seiner Ausführungsform 1; und
b) Einbringen des implantierbaren elektrischen medizinischen Geräts in einen eukaryotischen Organismus.

Ein bevorzugtes Einbringen ist dabei ein Implantieren. Ein bevorzugter eukaryotischer Organismus ist ein Mensch oder ein Tier oder beides. Ein bevorzugtes Einbringen erfolgt in eines ausgewählt aus der Gruppe bestehend aus einem Brustkorb, einem Herz, einem Thorax, einem Bauchraum, einem Abdomen, einem Kopf, einem Schädel, und einem Innenohr, oder in eine Kombination aus mindestens zwei davon.

Einen Beitrag zur Erfüllung mindestens einer der erfindungsgemäßen Aufgaben leistet eine Ausführungsform 1 einer Verwendung 2 eines implantierbaren elektrischen medizinischen Geräts 1 nach seiner Ausführungsform 1 oder eines implantierbaren elektrischen medizinischen Geräts 2 nach seiner Ausführungsform 1 in einer Therapie einer Krankheit oder einer Fehlfunktion oder beidem.

### Rahmen

Ein Rahmen ist erfindungsgemäß bevorzugt ein Torus, torusförmig, oder ein Prisma, wobei das Prisma eine erste Grundfläche und eine zweite Grundfläche und mindestens einen Hohlraum beinhaltet, wobei der Hohlraum eine Teilfläche der ersten Grundfläche und eine Teilfläche der zweiten Grundfläche beinhaltet. Der Hohlraum ist eine Rahmenöffnung. Eine bevorzugte erste Grundfläche oder eine bevorzugte zweite Grundfläche oder beide sind eines ausgewählt aus der Gruppe bestehend aus einer Kreisfläche, einer Ellipsenfläche, einer Ovalfläche, einer Dreiecksfläche, einer Vierecksfläche, einer Fünfecksfläche, einer Sechsecksfläche, einer Siebenecksfläche, einer Achtecksfläche, einer Polygonfläche oder eine Kombination von mindestens zwei davon. Die Teilfläche der ersten Grundfläche oder die Teilfläche der zweiten Grundfläche oder beide, die die Rahmenöffnung beinhaltet sind bevorzugt eines ausgewählt aus der Gruppe bestehend aus einer Kreisfläche, einer Ellipsenfläche, einer Ovalfläche, einer Dreiecksfläche, einer Vierecksfläche, einer Fünfecksfläche, einer Sechsecksfläche, einer Siebenecksfläche, einer Achtecksfläche, einer Polygonfläche oder eine Kombination von mindestens zwei davon. Eine bevorzugte Polygonfläche ist eine Fläche eines regelmäßigen Polygons oder eine Fläche eines unregelmäßigen Polygons. Ein ganz bevorzugter Rahmen ist ein Hohlzylinder oder ein Ring oder beides. Ein weiterhin bevorzugter Rahmen ist eine Lochplatte. Eine Lochplatte ist eine Platte, beinhaltend eine Pluralität von sich gegenüberliegenden Flächen verbindenden Löchern. Ein erfindungsgemäß bevorzugter erster Rahmen ist ein Flansch. Ein weiterer bevorzugter erster Rahmen besteht aus einem Metall. Ein erfindungsgemäß bevorzugter weiterer Rahmen ist eine Durchführungshülse. Ein weiterer bevorzugter weiterer Rahmen besteht aus einer Keramik.

### erstes Bauteil

Ein erfindungsgemäß bevorzugtes erstes Bauteil ist ein Durchführungselement oder ein Anschlussstift, auch Anschlusspin genannt, oder beides. Ein weiteres bevorzugtes erstes Bauteil besteht aus einem Cermet.

### Metall für Gehäuse bzw. ersten Rahmen

Für das Metall aus dem das Gehäuse oder der erste Rahmen oder beide, bevorzugt beide, erfindungsgemäß bestehen kommen alle dem Fachmann geläufigen Metalle in Betracht, die neben einer Leitfähigkeit auch eine gute Verträglichkeit für eukaryotisches Gewebe aufweisen. Hierbei ist ein erfindungsgemäß bevorzugtes Metall vorzugsweise ausgewählt aus der Gruppe bestehend aus Platin, Iridium, Niob, Palladium, Eisen, Edelstahl, einer Kobalt-Chrom-Legierung, Molybdän, Tantal, Wolfram, Titan, Kobalt und Zirkonium oder aus einer Kombination von mindestens zwei davon. Dabei ist eine bevorzugte Kombination eine Legierung. Ein bevorzugter Edelstahl ist ein Edelstahl 316L. Ein bevorzugtes Metall ist biokompatibel. Eine bevorzugte Legierung ist biokompatibel.

### biokompatibles Material

Ein bevorzugtes biokompatibles Material ist eines ausgewählt aus der Gruppe bestehend aus biotolerant, bioinert und bioaktiv oder eine Kombination aus mindestens zwei davon.

### eukaryotisches Gewebe

Ein bevorzugtes eukaryotisches Gewebe ist ein tierisches Gewebe oder ein menschliches Gewebe oder beides.

### Cermet

Erfindungsgemäß wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix oder beides bezeichnet. Zur Herstellung eines Cermets kann beispielsweise ein Gemisch aus mindestens einem keramischen Pulver und mindestens einem metallischen Pulver verwendet werden, welches beispielsweise mindestens mit einem Bindemittel und ggf. mindestens mit einem Lösungsmittel versetzt werden kann. Das bzw. die keramischen Pulver des Cermets weisen vorzugsweise eine mittlere Korngröße von weniger als 10 µm, bevorzugt weniger als 5 µm, besonders bevorzugt weniger als 3 µm auf. Das bzw. die metallischen Pulver des Cermets wiesen vorzugsweise eine mittlere Korngröße von weniger als 15 µm, bevorzugt weniger als 10 µm, besonders bevorzugt weniger als 5 µm auf. Als mittlere Korngröße wird dabei insbesondere der Medianwert oder D₅₀-Wert der Korngrößenverteilung angesehen. Der D₅₀-Wert beschreibt jenen Wert, bei dem 50 % der Körner des keramischen Pulvers und/oder des metallischen Pulvers feiner sind als der D₅₀-Wert. Ein bevorzugtes Cermet weist eine hohe spezifische Leitfähigkeit auf, die bevorzugt mindestens 1 S/m, bevorzugter mindestens 100 S/m, bevorzugter mindestens 10³ S/m, bevorzugter mindestens 10⁴ S/m , noch bevorzugter mindestens 10⁵ S/m, und am bevorzugtesten mindestens 10⁶ S/m beträgt.

Die mindestens eine keramische Komponente eines erfindungsgemäßen Cermets beinhaltet bevorzugt eine erfindungsgemäße Keramik. Die mindestens eine metallische Komponente eines erfindungsgemäßen Cermets beinhaltet bevorzugt eines ausgewählt aus der Gruppe bestehend aus Platin, Iridium, Niob, Palladium, Eisen, Edelstahl, einer Kobalt-Chrom-Legierung, Molybdän, Tantal, Wolfram, Titan, Kobalt und Zirkonium oder Kombination von mindestens zwei davon. Dabei ist eine bevorzugte Kombination eine Legierung. Ein bevorzugter Edelstahl ist ein Edelstahl 316L. Eine elektrisch leitfähige Verbindung stellt sich im Cermet in der Regel dann ein, wenn der Metallgehalt über der sogenannten Perkolationsschwelle liegt, bei der die Metallpartikel im gesinterten Cermet mindestens punktuell miteinander verbunden sind, so dass eine elektrische Leitung ermöglicht wird. Dazu sollte der Metallgehalt erfahrungsgemäß, abhängig von der Materialauswahl, mindestens 25 Vol.-% betragen, bevorzugt mindestens 32 Vol.-%, am bevorzugtesten mindestens 38 Vol.-%, jeweils bezogen auf das gesamte Volumen des Cermets.

### Keramik

Eine erfindungsgemäße Keramik kann jede Keramik sein, die der Fachmann für den erfindungsgemäßen Einsatz auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid (Al₂O₃), Magnesiumoxid (MgO), Zirkoniumoxid (ZrO₂), Yttriumoxid (Y₂O₃), Aluminiumtitanat (Al₂TiO₅), einer Piezokeramik wie Blei-Zirkonat (PbZrO₃), Blei-Titanat (PbTiO₃) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon. Eine weitere bevorzugte Oxidkeramik beinhaltet eines ausgewählt aus der Gruppe bestehend aus Zirkoniumoxid verstärktes Aluminiumoxid (ZTA - Zirconia Toughened Aluminum - Al₂O₃/ZrO₂), Yttrium verstärktes Zirkoniumoxid (Y-TZP), Barium(Zr, Ti)oxid, Barium(Ce, Ti)oxid oder eine Kombination aus mindestens zwei davon.

Die Silikatkeramik ist bevorzugt ausgewählt aus der Gruppe bestehend aus einem Steatit (mg₃[Si₄O₁₀(OH)₂]), Cordierit (Mg, Fe²⁺)₂(Al₂Si)[Al₂Si₄O₁₈]), Mullit (Al₂Al₂₊₂ₓSi₂₋₂ₓO₁₀₋ₓ mit x = Sauerstoffleerstellen pro Elementarzelle), Feldspat (Ba,Ca,Na,K,NH₄)(Al,B,Si)₄O₈) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid (B₄C), Titancarbid (TiC), Wolframcarbid, Zementit (Fe3C). Das Nitrid kann ausgewählt sein aus der Gruppe bestehend aus Siliciumnitrid (Si₃N₄), Aluminiumnitrid (AlN), Titannitrid (TiN), Siliciumaluminiumoxinitrid (SIALON) oder einer Mischung aus mindestens zwei davon. Eine weitere bevorzugte Nichtoxid-Keramik ist Natrium-Kalium-Niobat.

### hermetisch dicht

Im Rahmen der Erfindung kann der Begriff "hermetisch dicht" verdeutlichen, dass bei einem bestimmungsgemäßen Gebrauch innerhalb üblicher Zeiträume (beispielsweise 5 bis 10 Jahre) Feuchtigkeit oder Gase oder beides nicht oder nur minimal durch eine hermetisch dichte Barriere hindurch ausgetauscht werden können. Eine physikalische Größe, die beispielsweise eine Permeation von Gasen oder Feuchtigkeit oder beidem durch die Barriere beschreiben kann, ist die sogenannte Leckrate, welche beispielsweise durch Lecktests bestimmt werden kann. Entsprechende Lecktests können beispielsweise mit Heliumlecktestern und/oder Massenspektrometern durchgeführt werden und sind im Standard Mil-STD-883G Method 1014 spezifiziert. Die maximal zulässige Helium-Leckrate wird dabei abhängig vom internen Volumen der zu prüfenden Vorrichtung festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, können diese maximal zulässigen Helium-Leckraten beispielsweise von 1 x 10⁻⁸ atm×cm³/s bis 1 x 10⁻⁷ atm×cm³/s betragen. Im Rahmen der Erfindung kann der Begriff "hermetisch dicht" insbesondere bedeuten, dass die Barriere, hier bevorzugt das Gehäuse, eine Helium-Leckrate von weniger als 1 x 10⁻⁷ atm×cm³/s aufweist. In einer vorteilhaften Ausführung kann die Helium-Leckrate weniger als 1 x 10⁻⁸ atm×cm³/s, insbesondere weniger als 1 x 10⁻⁹ atm×cm³/s betragen.

Zum Zweck der Standardisierung können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben. Aufgrund der Einsatzart von implantierbaren elektrischen medizinischen Geräten ist deren hermetische Dichtigkeit und Biokompatibilität in der Regel eine der vorrangigsten Anforderungen. Das hier vorgeschlagene implantierbare elektrische medizinische Gerät kann insbesondere in einen Körper eines menschlichen oder tierischen Benutzers, insbesondere eines Patienten, eingesetzt werden. Dadurch ist das Gehäuse in der Regel einer Flüssigkeit eines Körpergewebes des Körpers ausgesetzt. Somit ist es in der Regel von Bedeutung, dass weder Körperflüssigkeit in das Gehäuse eindringt, noch das Flüssigkeiten aus dem Gehäuse austreten. Um dieses sicherzustellen, sollte das Gehäuse eine möglichst vollständige Undurchlässigkeit aufweisen, insbesondere gegenüber Körperflüssigkeiten.

### implantierbares elektrisches medizinisches Gerät

Ein bevorzugtes implantierbares elektrisches medizinisches Gerät ist ein Therapiegerät. Ein bevorzugtes implantierbares Therapiegerät ist in einen Brustkorb oder einen Kopf, oder beides implantierbar. Ein weiteres bevorzugtes Therapiegerät ist ein Defibrillator oder ein Schrittmacher oder eine Kombination aus mindestens zwei davon. Ein bevorzugter Schrittmacher ist einer ausgewählt aus der Gruppe bestehend aus einem Herzschrittmacher, einem Blasenschrittmacher, einem Darmschrittmacher, einem Hirnschrittmacher, einem Atemschrittmacher und einem Zwerchfellschrittmacher oder eine Kombination aus mindestens zwei davon. Ein besonders bevorzugter Schrittmacher ist ein Herzschrittmacher. Ein bevorzugter Herzschrittmacher ist ein kabelloser Herzschrittmacher. Ein weiteres bevorzugtes Therapiegerät ist ein Cochleaimplantat.

Ein weiteres bevorzugtes implantierbares elektrisches medizinisches Gerät ist ein Diagnosegerät. Ein bevorzugtes Diagnosegerät ist ein Biomonitor. Ein bevorzugter Biomonitor beinhaltet eines ausgewählt aus der Gruppe bestehend aus einem EKG-Gerät, einem Holter-Monitor, einem Event-Recorder, und einem Loop-Recorder, oder eine Kombination von mindestens zwei davon. Ein bevorzugtes EKG-Gerät ist ein Langzeit-EKG-Gerät, welches bevorzugt Daten speichert, die über einen Zeitraum von mindestens einer Stunde anfallen. Ein weiteres bevorzugtes Diagnosegerät beinhaltet eine Sendeeinrichtung oder einen Datenspeicher oder beides. Eine bevorzugte Sendeeinrichtung ist ausgebildet zu einem drahtlosen, bevorzugt telemetrischen, Übertragen von Daten, bevorzugt EKG-Daten. Ein bevorzugtes drahtloses Übertragen von Daten ist ein Übertragen mittels Wellen. Bevorzugte Wellen sind Longitudinalwellen oder Transversalwellen oder beides. Bevorzugte Longitudinalwellen sind akustische Wellen oder Schallwellen oder beides. Bevorzugte Transversalwellen sind elektromagnetische Wellen. Bevorzugte elektromagnetische Wellen sind Wellen der Frequenz eines Mobilfunknetzes oder von Bluetooth oder beides. Ein bevorzugtes Mobilfunknetz ist ein GSM-Netz. Als Datenspeicher kann jede Einheit zum Speichern von Daten ausgewählt werden, welche der Fachmann für geeignet zum Speichern medizinischer Daten, bevorzugt EKG-Daten, in einem implantierbaren Gerät hält. Ein bevorzugter Datenspeicher ist ein Magnetspeicher oder ein Flashspeicher oder beides.

### Meniskus

Ein Meniskus ist eine Wölbung einer Oberfläche des dritten Bauteils, wobei die Wölbung durch eine Grenzflächenspannung einer flüssigen Phase hervorgerufen wurde. Eine bevorzugte flüssige Phase ist dabei eine Schmelze. Ein bevorzugter Meniskus ist ein konvexer Meniskus oder ein konkaver Meniskus oder beides.

### Zusammensetzung

Eine bevorzugte Zusammensetzung ist eine Paste. Eine bevorzugte Paste ist viskos. Eine weitere bevorzugte Paste weist eine Viskosität auf, die zu einem Drucken, bevorzugt einem Schablonendrucken, geeignet ist. Eine bevorzugte Flüssigkeit, beinhaltet von der Zusammensetzung, ist ein Vehikel.

### Metallpartikel der Zusammensetzung

Bevorzugte Metallpartikel, beinhaltet von der Zusammensetzung, bestehen aus einem ausgewählt aus der Gruppe bestehend aus Ag, Au, Cu, Pd, und Pt, oder einer Kombination aus mindestens zwei davon. Dem Fachmann ist bekannt, dass Metallpartikel verschiedene Formen, Oberflächenausgestaltungen, Größen, spezifische Oberflächen, und spezifische Oberfläche oder Oberflächeninhalt zu Volumen-Verhältnisse haben können. Viele verschiedene mögliche Formen von Metallpartikeln sind dem Fachmann bekannt. Beispielhaft gehören dazu sphärische, eckige, abgerundete, längserstreckte (stäbchenförmige und nadelförmige) und flache (flächenförmige) Formen. Die Metallpartikel können auch eine Mischung aus Teilchen verschiedener Formen beinhalten. Metallpartikel mit einer Form oder einer Kombination von Formen, die ein vorteilhaftes Sinterverhalten, einen elektrischen Kontakt, ein Haften, und eine elektrische Leitfähigkeit begünstigen sind bevorzugt. Die Metallpartikel verfügen über eine Länge, eine Breite und eine Dicke. Wird die Partikelgröße angegeben bezieht sich dies auf die Länge der Metallpartikel. Die Länge eines Partikels ist die Länge der längsten Geraden, welche Anfangs- und Endpunkt auf der Oberfläche des Partikels hat. Die Breite des Partikels ist die Länge der längsten Geraden, welche Anfangs- und Endpunkt auf der Oberfläche des Partikels hat und senkrecht zur Länge des Partikels ist. Die Dicke des Partikels ist die Länge der längsten Geraden, welche Anfangs- und Endpunkt auf der Oberfläche des Partikels hat und senkrecht sowohl zur Länge des Partikels als auch zu dessen Breite, jeweils gemäß obiger Definition, ist. In einer Ausgestaltung der Erfindung sind möglichst gleichförmige Metallpartikel bevorzugt. Darin sind die Verhältnisse von Länge, Breite und Dicke zueinander möglichst nahe 1 sind. Bevorzugt liegen diese Verhältnisse in einem Bereich von 0,7 bis 1,5, bevorzugter von 0,8 bis 1,3, am bevorzugtesten von 0,9 bis 1,2. In dieser Ausgestaltung sind beispielsweise sphärische oder würfelförmige Formen der Metallpartikel oder Kombinationen von beiden bevorzugt. In einer anderen Ausgestaltung der Erfindung sind möglichst ungleichförmige Metallpartikel bevorzugt. Dabei ist bevorzugt mindestens eines der Verhältnisse von Länge, Breite und Dicke zueinander größer als 1,5, bevorzugt größer als 3, bevorzugter größer als 5. Gemäß dieser Ausgestaltung sind flockenförmige, stäbchenförmige und nadelförmige Metallpartikel oder eine Kombination aus mindestens zwei der vorgenannten Metallpartikel oder eine Kombination mit einer anderen Metallpartikelform bevorzugt. Bevorzugt sind weiter Metallpartikel mit Oberflächen, welche ein effektives Sintern sowie einen guten elektrischen Kontakt und eine hohe elektrische Leitfähigkeit begünstigen.

### Vehikel

Bevorzugte Vehikel sind anorganische und organische Vehikel. Ein bevorzugtes anorganisches Vehikel ist Wasser. Ein bevorzugtes organisches Vehikel ist eine Lösung, eine Emulsion oder eine Dispersion beinhaltend eines oder mehrere Lösungsmittel. Dabei ist ein organisches Lösungsmittel bevorzugt, welches sicherstellt, dass die Bestandteile der Zusammensetzung in einer dispergierten, emulgierten oder gelösten Form vorliegen. Bevorzugte organische Vehikel begünstigen eine optimale Stabilität der Bestandteile in der Zusammensetzung und verleihen der Zusammensetzung eine Viskosität, welche ein effektives Drucken der Zusammensetzung ermöglicht. Bevorzugte erfindungsgemäße Vehikel beinhalten als Vehikelbestandteile:
(i) ein Bindemittel, bevorzugt in einem Bereich von 1 bis 50 Gew.-%, bevorzugter von 2 bis 45 Gew.-%, am bevorzugtesten von 3 bis 40 Gew.-%;
(ii) ein Tensid, bevorzugt in einem Bereich von 0 bis 10 Gew.-%, bevorzugter von 0 bis 8 Gew.-%, am bevorzugtesten von 0 bis 6 Gew.-%;
(iii) eines oder mindestens zwei Lösungsmittel, deren Anteile sich nach den Anteilen der anderen Vehikelbestandteile bestimmen;
(iv) optional Additive, bevorzugt in einem Bereich von 0 bis 10 Gew.-%, bevorzugter von 0 bis 8 Gew.-%, am bevorzugtesten von 0 bis 5 Gew.-%;
wobei die Gew.-% sich jeweils auf das Gesamtgewicht des Vehikels beziehen und zu 100 Gew.-% summieren. Erfindungsgemäß bevorzugte Vehikel verleihen der Zusammensetzung eine möglichst gute Eignung zum Drucken.

### Bindemittel

Bevorzugte Bindemittel sind solche, die zum Erhalten einer Zusammensetzung mit geeigneter Stabilität, Eignung zum Drucken, Viskosität und Sinterverhalten beitragen. Bindemittel sind dem Fachmann bekannt. Alle Bindemittel, welche dem Fachmann als geeignet für einen erfindungsgemäßen Einsatz erscheinen, können als Bindemittel in dem Vehikel eingesetzt werden. Bevorzugte Bindemittel sind Harze. Weitere bevorzugte Bindemittel sind polymerische Bindemittel, monomerische Bindemittel und Bindemittel aus einer Kombination aus Polymeren und Monomeren. Polymerische Bindemittel können auch Co-Polymere sein, wobei mindestens zwei monomerische Einheiten in einem einzelnen Molekül beinhaltet sind. Bevorzugte polymerische Bindemittel beinhalten eine funktionelle Gruppe in der Hauptkette des Polymers, außerhalb der Hauptkette, oder in der Hauptkette und außerhalb der Hauptkette. Bevorzugte Bindemittel mit einer funktionellen Gruppe in der Hauptkette sind Polyester, substituierte Polyester, Polycarbonate, substituierte Polycarbonate, Polymere mit einer cyclischen Gruppe in der Hauptkette, Polyzucker, substituierte Polyzucker, Polyurethane, substituierte Polyurethane, Polyamide, substituierte Polyamide, Phenolharze, substituierte Phenolharze, Co-Polymere der Monomere von einem oder mehreren der vorgenannten Polymeren, optional mit anderen CoMonomeren, oder eine Kombination aus mindestens zwei davon. Bevorzugte Polymere mit einer cyclischen Gruppe in der Hauptkette sind Polyvinylbutylate (PVB) und deren Derivate, und Poly-Terpineol und dessen Derivate, oder Mischungen daraus. Bevorzugte Polyzucker sind Zellulose und Alkylderivate davon, bevorzugt Methylzellulose, Ethylzellulose, Propylzellulose, Butylzellulose, und deren Derivate, und Mischungen aus mindestens zwei davon. Bevorzugte Polymere mit einer funktionellen Gruppe außerhalb ihrer Hauptkette sind solche mit einer Amidgruppe, mit einer Säuregruppe und/oder einer Estergruppe (auch Acrylharze genannt), oder Polymere mit einer Kombination der vorgenannten funktionellen Gruppen. Bevorzugte Polymere mit einer Amidgruppe außerhalb der Hauptkette sind Polyvinyl-Pyrolidon (PVP) und dessen Derivate. Bevorzugte Polymer mit einer Säuregruppe und/oder einer Estergruppe außerhalb der Hauptkette sind Polyacrylsäure und ihre Derivate, Polymethacrylat (PMA) und dessen Derivate, und Polymethylmethacrylat (PMMA) und dessen Derivate, oder Kombinationen aus mindestens zwei davon. Bevorzugte monomerische Bindemittel sind ethylenglycolbasierte Monomere, Terpineol-Harze, und Harzderivate, oder eine Kombination aus mindestens zwei davon. Bevorzugte ethylenglycolbasierte monomerische Bindemittel haben eine Ethergruppe, ein Estergruppe, oder beides. Bevorzugte Ethergruppen sind dabei Methyl-, Ethyl-, Propyl-, Butyl-, Pentyl-, Hexyl-, und höhere Alkylethergruppen. Bevorzugte Estergruppen sind Acetat und dessen Alkylderivate, bevorzugt Ethylenglycol-Monobutylether-Monoacetat, oder eine Mischung aus den Vorgenannten. Besonders bevorzugte Bindemittel sind Alkylzellulose, bevorzugt Ethylzellulose, und deren Derivate, und Mischungen daraus mit anderen Bindemitteln ausgewählt aus den oben aufgeführten oder anderen.

### Tensid

Bevorzugte Tenside sind solche, die zum Erhalten einer Zusammensetzung mit geeigneter Stabilität, Eignung zum Drucken, Viskosität und Sinterverhalten beitragen. Tenside sind dem Fachmann bekannt. Alle Tenside, welche dem Fachmann als geeignet für einen erfindungsgemäßen Gebrauch erscheinen, können als Tensid in dem Vehikel eingesetzt werden. Bevorzugte Tenside basieren auf linearen Ketten, verzweigten Ketten, aromatischen Ketten, fluoridierten Ketten, Siloxanketten, Polyetherketten, oder Kombinationen aus mindestens zwei davon. Bevorzugte Tenside sind einfachkettig, doppelkettig, oder polykettig. Bevorzugte Tenside haben nichtionische, anionische, kationische, oder zwitterionische Enden. Weitere bevorzugte Tenside sind polymerisch oder monomerisch oder eine Mischung aus beidem. Bevorzugte Tenside können pigmentaffine Gruppen haben, bevorzugt hydroxyfunktionale Carbonsäureester mit pigmentaffinen Gruppen (zum Beispiel DISPERBYK®-108, hergestellt von BYK USA, Inc.), Acrylatco-polymere mit pigmentaffinen Gruppen (zum Beispiel DISPERBYK®-116, hergestellt von BYK USA, Inc.), modifizierte Polyether mit pigmentaffinen Gruppen (zum Beispiel TEGO® DISPERS 655, hergestellt von Evonik Tego Chemie GmbH), und andere Tenside mit Gruppen hoher Pigmentaffinität (zum Beispiel TEGO® DISPERS 662 C, hergestellt von Evonik Tego Chemie GmbH). Andere bevorzugte Polymere sind Polyethylenglycol und seine Derivate, und Alkylcarbonsäure und ihre Derivate oder Salze, oder Mischungen aus mindestens zwei davon. Ein bevorzugtes Polyethylenglycol ist Poly(ethylenglycol-)essigsäure. Bevorzugte Alkylcarbonsäuren sind solche mit völlig gesättigten oder einfach oder mehrfach ungesättigten Alkylketten oder Mischungen aus mindestens zwei davon. Bevorzugte Carbonsäuren mit gesättigten Alkylketten sind solche mit Alkylkettenlängen in einem Bereich von 8 bis 20 Kohlenstoffatomen, bevorzugt C₉H₁₉COOH (Caprinsäure), C₁₁H₂₃COOH (Laurinsäure), C₁₃H₂₇COOH (Myristinsäure), C₁₅H₃₁COOH (Palmitinsäure), C₁₇H₃₅COOH (Stearinsäure), oder Mischungen aus mindestens zwei davon. Bevorzugte Carbonsäuren mit ungesättigten Alkylketten sind C₁₈H₃₄O₂ (Ölsäure) und C₁₈H₃₂O₂ (Linolsäure).

### Lösungsmittel

Bevorzugte Lösungsmittel sind solche, die in dem Verfahrensschritt f) oder g) oder in beiden des erfindungsgemäßen Verfahrens 1 zu einem wesentlichen Anteil aus der Zusammensetzung entfernt werden können. Bevorzugt wird in dem Verfahrensschritt f) oder g) oder in beiden der Gewichtsanteil des Lösungsmittels an der Zusammensetzung um 80 %, bevorzugt um 95 %, basierend auf dem Gewichtsanteil unmittelbar vor Verfahrensschritt f) verringert. Bevorzugte Lösungsmittel sind solche, die zum Erhalten einer Zusammensetzung mit geeigneter Stabilität, Eignung zum Drucken, Viskosität und Sinterverhalten beitragen und welche zu einer geeigneten elektrischen Leitfähigkeit und elektrischen Kontakt des dritten Bauteils führen. Lösungsmittel sind dem Fachmann bekannt. Alle Lösungsmittel, welche dem Fachmann als geeignet für einen erfindungsgemäßen Gebrauch erscheinen, können als Lösungsmittel in dem Vehikel eingesetzt werden. Erfindungsgemäß bevorzugt sind Lösungsmittel, die es ermöglichen, der Zusammensetzung eine möglichst gute Eignung zum Drucken zu verleihen. Bevorzugte Lösungsmittel sind bei Standardraumtemperatur (298,15 K, 100 kPa) flüssig. Weitere bevorzugte Lösungsmittel haben eine Siedetemperatur über 90°C oder eine Schmelztemperatur über -20°C oder beides. Weitere bevorzugte Lösungsmittel sind polar oder unpolar, protisch, aprotisch, aromatisch, nicht-aromatisch, ionisch, oder eine Kombination aus mindestens zwei davon. Bevorzugte Lösungsmittel sind Monoalkohole, Dialkohole, Polyalkohole, Monoester, Diester, Polyester, Monoether, Diether, Polyether, Lösungsmittel beinhaltend eine oder mindestens zwei funktionelle Gruppen dieser Kategorien, optional beinhaltend funktionelle Gruppen anderer Kategorien, bevorzugt cyclische Gruppen, aromatische Gruppen ungesättigte Bindungen, Alkoholgruppen in denen ein oder mindestens zwei Sauerstoffatome ersetzt sind durch Heteroatome, Ethergruppen in denen ein oder mindestens zwei Sauerstoffatome ersetzt sind durch Heteroatome, Estergruppen in denen ein oder mindestens zwei Sauerstoffatome ersetzt sind durch Heteroatome, und Mischungen aus mindestens zwei davon. Dabei sind bevorzugte Ester Dialkylester von Adipinsäure, mit bevorzugten Alkylbestandteilen Methyl-, Ethyl-, Propyl-, Butyl-, Pentyl-, Hexyl-, und höhere Alkylgruppen oder Mischungen aus mindestens zwei davon, bevorzugt Dimethyladipin, und Mischungen aus zwei oder mehr Adipinestern. Bevorzugte Ether sind Diether, bevorzugt Dialkylether von Ethylenglycol, mit bevorzugten Alkylbestandteilen Methyl-, Ethyl-, Propyl-, Butyl-, Pentyl-, Hexyl-, und höheren Alkylgruppen oder Mischungen aus mindestens zwei davon, und Mischungen aus zwei Diethern. Bevorzugte Alkohole sind primäre, sekundäre und tertiäre Alkohole, oder eine Mischung aus zwei oder mehr Alkoholen. Ein bevorzugter Alkohol ist Terpineol und seine Derivate. Bevorzugte Alkohole, welche zwei oder mehr funktionelle Gruppen beinhalten sind 2,2,4-Trimethyl-1,3-Pentandiol-Mono-Iso-Butyrat, auch Texanol genannt, und dessen Derivate, 2-(2-Ethoxyethoxy)Ethanol, auch Carbitol genannt, dessen Alkylderivate, bevorzugt Methyl-, Ethyl-, Propyl-, Butyl-, Pentyl-, Hexylcarbitol, bevorzugt Hexylcarbitol oder Butylcarbitol, und Acetatderivate dessen, bevorzugt Butylcarbitolacetat, oder Mischungen aus mindestens zwei der Vorgenannten.

### Additive

Bevorzugte Additive in dem Vehikel sind von den vorgenannten Vehikelbestandteilen verschieden und tragen zu bevorzugten Eigenschaften der Zusammensetzung bei, wie geeignete Viskosität, Sintereigenschaften, elektrische Leitfähigkeit des aus der Zusammensetzung hergestellten dritten Bauteils, elektrischer Kontakt. Alle Additive, welche dem Fachmann als geeignet für einen erfindungsgemäßen Einsatz erscheinen, können als Additive in dem Vehikel eingesetzt werden. Erfindungsgemäß bevorzugte Additive sind Thixotropiermittel, Viskositätsregulatoren, Stabilisierungsmittel, anorganische Additive, Verdickungsmittel, Emulgiermittel, Dispergiermittel, und pH-regulatoren. Bevorzugte Thixotropiermittel sind Carbonsäurederivate, bevorzugt Fettsäurederivate. Bevorzugte Fettsäurederivate sind C₉H₁₉COOH (Caprinsäure), C₁₁H₂₃COOH (Laurinsäure), C₁₃H₂₇COOH (Myristinsäure), C₁₅H₃₁COOH (Palmitinsäure), C₁₇H₃₅COOH (Stearinsäure), C₁₈H₃₄O₂ (Ölsäure) und C₁₈H₃₂O₂ (Linolsäure), oder Mischungen aus mindestens zwei davon. Eine bevorzugte Mischung, beinhaltend Fettsäurederivate, ist Castoröl.

### Überlagern

Die Zusammensetzung kann auf jede dem Fachmann bekannte Art, die zum erfindungsgemäßen Überlagern einer Kontaktoberfläche mit der Zusammensetzung geeignet erscheint, überlagert, bevorzugt auf die Kontaktoberfläche aufgetragen, werden. Dies beinhaltet, ist aber nicht beschränkt auf, Imprägnieren, Dippen, Tropfen, Gießen, Spritzen, Sprühen, Rakeln, Gießlackieren, Streichen, Drucken, oder eine Kombination aus mindestens zwei davon. Bevorzugte Druckverfahren sind Strahldrucken, Siebdrucken, Tampondrucken, Offsetdrucken, Reliefdrucken und Schablonendrucken oder eine Kombination aus mindestens zwei davon. Besonders bevorzugt ist Drucken, ganz besonders Schablonendrucken.

### Therapie / Krankheit / Fehlfunktion

Eine bevorzugte Therapie ist eine Neurotherapie oder eine Herztherapie oder beides. Hierbei beinhaltet eine Neurotherapie bevorzugt eine elektrische Stimulation eines Nervs. Eine bevorzugte Herztherapie beinhaltet bevorzugt eine elektrische Stimulation eines Herzens. Eine bevorzugte Neurotherapie ist eine ausgewählt aus der Gruppe bestehend aus einer tiefen Hirnstimulation (Deep Brain Stimulation - DBS), einer Rückenmarksstimulation, einer Vagusnervstimulation, einer Sacralnervstimulation und einer Magen -oder Darmnervenstimulation oder eine Kombination aus mindestens zwei davon. Eine bevorzugte Herztherapie ist eine ausgewählt aus der Gruppe bestehend aus einer Herzschrittmachertherapie, einer ICD-Therapie, einer CRT-P-Therapie und einer CRT-D-Therapie oder eine Kombination aus mindestens zwei davon. Eine bevorzugte tiefe Hirnstimulation ist eine Therapie von einem ausgewählt aus der Gruppe bestehend aus Alzheimer, einer Parkinson-Krankheit, einem Tremor, einer Depression, einer Epilepsie, einer Dystonie, einer Zwangsstörung, einem Tourettesyndrom, einem Koma und einem Trauma oder eine Kombination aus mindestens zwei davon. Eine bevorzugte Rückenmarksstimulation ist eine Therapie von einem ausgewählt aus der Gruppe bestehend aus chronischem Rückenschmerzen, einem Postdiskotomiesyndrom, einem komplexen regionalen Schmerzsyndrom und einem therapieresistenten Schmerz, bevorzugt durch Ischämie, oder eine Kombination aus mindestens zwei davon. Eine bevorzugte Vagusnervstimulation ist eine Therapie von einem ausgewählt aus der Gruppe bestehend aus einer Epilepsie, einer Depression und einem chronischen Herzfehler (Chronic Heart Failure - CHF) oder eine Kombination aus mindestens zwei davon. Eine bevorzugte Sacralnervstimulation ist eine Therapie von einem ausgewählt aus der Gruppe bestehend aus einer Harninkontinenz, einem Harnverhalt, einem häufigen Harndrang, einer Stuhlinkontinenz, einer idiopathischen Obstipation, einer interstitiellen Cystitis und einer chronischen Analfissur oder eine Kombination aus mindestens zwei davon. Eine bevorzugte Magen -oder Darmnervenstimulation ist eine Therapie von Fettleibigkeit. Eine bevorzugte Herzschrittmachertherapie ist eine Therapie von Bradykardie oder Tachykardie oder beidem. Eine bevorzugte ICD-Therapie ist eine Therapie von Kammerflimmern oder ventrikulärer Tachykardie oder beidem. Eine bevorzugte CRT-P-Therapie ist eine Therapie eines chronischen Herzfehlers. Eine bevorzugte CRT-D-Therapie ist eine Therapie einer herzfehlerinduzierten Leitungsstörung oder einer ventrikulären Dyssynchronie oder beidem.

### MESSMETHODEN

Die folgenden Messmethoden wurden im Rahmen der Erfindung benutzt. Sofern nichts anderes angegeben ist wurden die Messungen bei einer Umgebungstemperatur von 25°C, einem Umgebungsluftdruck von 100 kPa (0,986 atm) und einer relativen Luftfeuchtigkeit von 50 % durchgeführt.

### Biokompatibilität

Die Biokompatibilität wird gemäß der Norm nach 10993-4:2002 bestimmt.

### Rauheit

Die Rauheit wird ermittelt gemäß der Norm EN ISO 4288 : 1997 als die darin beschriebene mittlere Rauheit Rₐ.

### Porosität

Zur Messung der Porosität wurden zunächst metallographische Schliffproben durch Einbetten in Epoxidharz, Schleifen mit SiC-Papier mit sukzessive kleinerer Körnung sowie Polieren mit einer Diamantpaste hergestellt. Die äußerste Schicht jeder Probe wurde durch Ätzen der Probe abgetragen. Im Folgenden wurden Aufnahmen der so behandelten Probenoberfläche mit einem Lichtmikroskop und einem Elektronenmikroskop gemacht. Hierbei sollte ein möglichst hoher Kontrast zwischen den Poren der Probe und dem Material (Metall und Keramik) erzielt werden. Zur Auswertung der Bilder wurden diese Graustufenbilder mittels Otsu-Methode in binäre Bilder umgewandelt. Das heißt, die Bildpixel wurden mittels eines Schwellenwertes jeweils einer Pore oder dem Probenmaterial zugeordnet. Anschließend wurde die Porosität an Hand der binären Bilder als Quotient aus der Anzahl der Pixel, welche Poren darstellen und der Gesamtanzahl der Pixel pro Bild ermittelt. Hierbei wurde die Porosität als arithmetischer Mittelwert aus 5 Bildern, jeweils aufgenommen an 5 Schliffproben, bestimmt.

### Rohdichte / Reindichte

Das Verhältnis von Rohdichte zu Reindichte berechnet sich aus Porosität = 1 - (Rohdichte / Reindichte). Die Rohdichte ist die tatsächliche Dichte der Probe mit Poren. Die Reindichte ist die Dichte der Probe ohne Poren.

### elektrischer Widerstand des dritten Bauteils / der elektrisch leitenden Verbindung

Der elektrische Widerstand wird durch eine Vierleitermessung ermittelt. Bei der Vierleiter-Messanordnung fließt über zwei der Leitungen ein bekannter elektrischer Strom durch den Widerstand. Die am Widerstand abfallende Spannung wird hochohmig über zwei weitere Leitungen abgegriffen und mit einem Spannungsmessgerät gemessen. Der zu messende Widerstand wird daraus nach dem ohmschen Gesetz als Quotient aus Spannung und Stromstärke berechnet.

### metallographisches Schliffbild

Die Probe wird mit einer mit einer Diamantscheibe ausgestatteten und gekühlten Trennsäge so geschnitten, dass die zu untersuchende Fläche offengelegt wird. Die geschnittene Probe wird in einen Behälter, welcher mit EpoFix (Struers GmbH) als Einbettungsmaterial gefüllt ist, gelegt. Das Einbettungsmaterial wurde zuvor gemäß den Benutzungshinweisen angemischt. Nach 8-stündigen Aushärten bei Raumtemperatur kann die Probe weiterverarbeitet werden. Zunächst wird die Probe mit einer Labopol-25 (Struers GmbH) geschliffen. Hierbei wird Siliciumcarbidpapier 180-800 (Struers GmbH) bei 250 Umdrehungen pro Minute verwendet. Im Weiteren wird die Probe mit einer Rotopol-2, ausgestattet mit Retroforce-4, MD Piano 220 und MD allegro, poliert. Das erhaltene Schliffbild wird mit einem Rasterelektronenmikroskop Zeiss Ultra 55 (Carl Zeiss AG), ausgestattet mit einer Feldemissionselektrode, bei einer Beschleunigungsspannung von 20 kV und einem Druck von ca. 3×10⁻⁶ mbar untersucht. In einigen Fällen wurde das Schliffbild verwendet um die Elementzusammensetzung des Probenmaterials zu bestimmen. Hierzu wurde eine EDX-Messung (energy dispersive X-ray spectroscopy) als Linienscan durchgeführt. Hierzu wurde das Zeiss Ultra 55 mit IncaPentaFETx3 und der Software "The Microanalysis Suite Issue 18d + SP3" (beides von Oxford Instruments) sowie eine Apertur von 30 µm eingesetzt.

### Korngröße

Die Korngröße wird anhand eines nach der obigen Methode erhaltenen metallographischen Schliffbildes nach dem Linienschnittverfahren ermittelt. Dabei werden Linienmuster verwendet und die Korngröße auf der Grundlage der mittleren Schnittlänge ermittelt. Das Verfahren wird gemäß EN 623-3:2003 durchgeführt. Somit ist die hierin verwendete Korngröße gleich der in der Norm gemessenen mittleren, aus der Schnittlinienlänge bestimmten Korngröße. Für die Messung wird weiterhin das Rasterelektronenmikroskop Zeiss Ultra 55 (Carl Zeiss AG) verwendet. Gemäß Punkt 7.3 der Norm werden für jede Messung Mikrogefügeaufnahmen von 3 verschiedenen Bereichen der Probe gemacht. Ferner wird im Falle der Anwendbarkeit des Verfahrens A (Kriterien siehe Norm) nach der ersten Alternative unter Punkt 8.2 der Norm verfahren.

### Porengröße

Die Porengröße wird anhand eines nach der obigen Methode erhaltenen metallographischen Schliffbildes nach dem Linienschnittverfahren ermittelt. Dabei werden Linienmuster verwendet und die Porengröße auf der Grundlage der mittleren Schnittlänge ermittelt. Das Verfahren wird analog der Norm EN 623-3:2003 für Korngrößen durchgeführt. Hierbei wird das unter Punkt 8.3 beschriebene Verfahren mit der Auswertung nach Punkt 9.2, wobei jeweils auf die Poren abzustellen ist, angewendet.

### massenspezifische Oberfläche

Die massenspezifische Oberfläche wird gemäß DIN ISO 9277:2003-05 durch Gasadsorption nach dem BET-Verfahren bestimmt. Hierbei wird Stickstoff als Adsorbtiv verwendet. Bei der Entgasung wird ein Kontrollzeitraum, über den sich der Druck im Wesentlichen nicht ändert, von 20 Minuten verwendet. Der Sättigungsdampfdruck des Adsorbtivs wird direkt mit einem Stickstoffdampfdruckthermometer gemessen. Es wird das gravimetrische Messverfahren nach Punkt 6.3.2 der Norm unter Beachtung der gegebenen Sollvorschriften angewendet. Zur Auswertung wird von der Gleichung (1) unter Punkt 7.1 ausgegangen. Es wird die Mehrpunktbestimmung nach Punkt 7.2 angewendet. Wie in der Norm empfohlen wird für Stickstoff bei 77 K der Flächenbedarf aₘ= 0,162 nm² verwendet. Die Einpunktbestimmung nach 7.3 wird nicht verwendet. Zur Messung wird ein "Gemini 2360 Surface Area Analyzer" der Micrometrics GmbH (Rutherford 108, D-52072 Aachen) eingesetzt.

### Abstand erste und weitere Kontaktoberfläche

Der Abstand zwischen erster und weiterer Kontaktoberfläche wird mit einer Bügelmessschraube gemessen.

### D₅₀ der Metallpartikel

Die Messung der Partikelgröße (D₅₀) erfolgt gemäß ISO 13317-3:2001. Ein Sedigraph 5100 mit der Software Win 5100 V2.03.01 (von Micromeritics) wird für die Messung verwendet. Das Gerät arbeitet mit Röntgenstrahlung und gravitativer Sedimentation. Eine Probe von 400 bis 600 mg der zu vermessenden Metallpartikel wird abgewogen in einem 50 ml-Becherglas und 40 ml Sedisperse P11 (von Micromeritics, Dichte ca. 0,74 bis 0,76 g/cm³, Viskosität ca. 1,25 bis 1,9 mPa·s) werden als Suspensiermittel zugefügt und somit eine Suspension erhalten. Ein Magnetrührfischchen wird hinzugegeben. Die Probe wird mit einem Ultrasonic Probe Sonifer 250 (von Branson) bei Power Level 2 für 8 Minuten homogenisiert. Dabei wird die Probe mit dem Magnetrührer gerührt. Die so vorbehandelte Probe wird in das Messinstrument gegeben und die Messung gestartet. Die Temperatur der Suspension wird aufgezeichnet, wobei die Werte typischerweise im Bereich von 24 bis 45°C liegen. Für die Berechnung wird die gemessene Viskosität der Suspension bei dieser Temperatur verwendet. Die Dichte (Dichte von Silber 10,5 g/cm³) sowie die Masse der Probe werden verwendet, um die Partikelgröße zu bestimmen und D₅₀ anzugeben.

### Halogengehalt

Der Halogengehalt der Zusammensetzung wurde gemäß der Norm BS EN 14582 bestimmt.

### Schichtdicke

Zur Messung der Schichtdicke wird ein Profilometer Hommel-Etamic-Tester T8000 mit der Software Turbo-Wave V7.53 (beides Jenoptik) eingesetzt. Die Sonde TKLT 300/17 wird in einem Abstand von 2.5 mm bei einer Geschwindigkeit von 0.5 mm/s über die Schichtoberfläche geführt. Der Scan wird senkrecht zum Rand der Schicht durchgeführt. 32 solcher Scans wurden durchgeführt und als Ergebnis daraus der Mittelwert gebildet.

### Viskosität

Viskositätsmessungen werden mit einem Thermo Fischer Scientific Corp. "Haake Rheostress 6000" mit einer Grundplatte MPC60 Ti und einer Kegelplatte C 20/0,5° Ti sowie der Software "Haake RheoWin Job Manager 4.00.0003" durchgeführt. Nachdem der Abstandsnullpunkt eingestellt wurde, wird eine für die Messung ausreichende Probe auf die Grundplatte platziert. Die Kegelplatte wird in Messposition bewegt mit einem Abstand von 0.026 mm und überschüssiges Probenmaterial wird mit einem Spatel entfernt. Die Probe wird für drei Minuten auf 25°C temperiert und die Rotationsmessung gestartet. Die Scherrate wird von 0 auf 20 s⁻¹ in 48 s und 24 äquidistanten Messpunkten und weiter auf 150 s⁻¹ in 312 s und 156 äquidistanten Messpunkten erhöht. Nach einer Wartezeit von 60 s bei einer Scherrate von 150 s⁻¹ wird die Scherrate von 150 s⁻¹ auf 20 s⁻¹ in 312 s und 156 äquidistanten Messpunkten und weiter auf 0 in 48 s und 24 äquidistanten Messpunkten verringert. Mikrodrehmomentenkorrektur, Mikrostresskontrolle und Massenträgheitskontrolle waren aktiviert. Die gemessene Viskosität ist der Wert bei der Scherrate von 100 s⁻¹ während des Verringerns der Scherrate.

Die Erfindung wird im Folgenden durch Beispiele und Zeichnungen genauer dargestellt, wobei die Beispiele und Zeichnungen keine Einschränkung der Erfindung bedeuten.

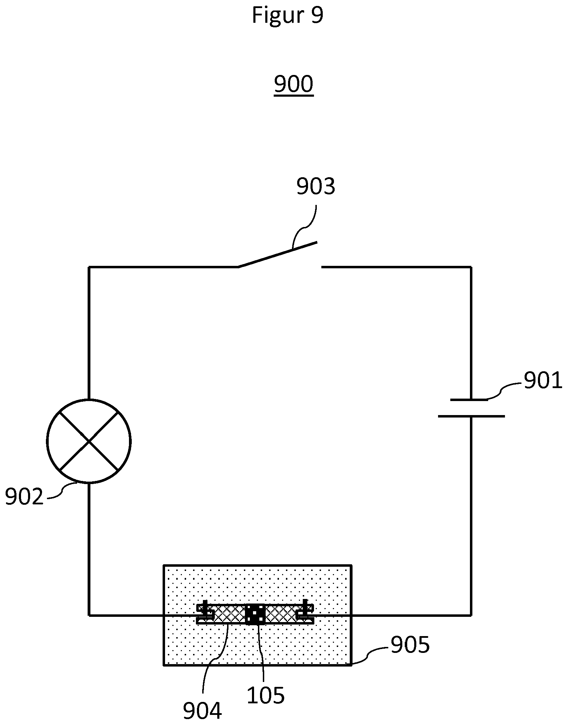

Für die erfindungsgemäßen Beispiele sowie die nicht erfindungsgemäßen Vergleichsbeispiele wurde jeweils ein Stromkreis gemäß Figur 9 aufgebaut. Der Stromkreis beinhaltete jeweils als Spannungsquelle eine 9 V-550 mAh-Alkali-Batterie des Herstellers Varta, sowie als Verbraucher in dem Stromkreis einen Elektromotor Typ 0816K009SR der Firma Faulhaber. Die Batterie wurde mittels Batterieclip und Kabeln mit dem Motor verbunden. Ferner beinhaltete jeder Stromkreis einen Schalter über den der Stromkreis unterbrochen werden konnte. Weiter wurde jeweils ein abisoliertes Kabelende in eine longitudinale Sackloch-Bohrung jeweils eines Kupferstiftes eingeführt und mittels einer transversal angreifenden Schraube fixiert. Die beiden Kupferstifte waren jeweils 10 mm lang und hatten einen Durchmesser von 2 mm. Die beiden freien Stirnflächen der Kupferstifte wurden je nach Beispiel und Vergleichsbeispiel durch eine nichterfindungsgemäße Lötverbindung oder durch eine erfindungsgemäße Sinterverbindung elektrisch miteinander verbunden. In jedem Fall hatte die elektrische Verbindung einen Durchmesser von 2 mm und eine Länge von 2 mm. Dies wurde durch Abschleifen in einem Nachbearbeitungsschritt nach dem Herstellen der Verbindung sichergestellt. Ferner wurden die Kupferstifte zusammen mit der elektrischen Verbindung jeweils mit einer Schraubklemme auf einem Elektrorütteltisch der FORM+TEST Seidner&Co. GmbH fixiert.

In den Beispielen und Vergleichsbeispielen wurde der Stromkreis mit dem Schalter geschlossen, so dass der Elektromotor im Leerlauf betrieben wurde. Gleichzeitig wurde der Rütteltisch bei 3000 Schwingungen pro Minute aktiviert. Nach einer Rütteldauer von 1 h wurde der Rütteltisch ausgeschaltet. Hatte sich innerhalb der Rütteldauer die zu prüfende elektrische Verbindung zwischen den beiden Kupferstiften gelöst, so dass der Motor stehen geblieben war, wurde dies als Ausfall gezählt. Lag kein Ausfall vor, wurde der Motor so lange betrieben bis die Spannungsquelle versagte. Die Dauer der Motorlaufzeit wurde gemessen und bestimmte die Batterielebensdauer. Für die einzelnen Beispiele und Vergleichsbeispiele sind die Details zur Herstellung der elektrischen Verbindung zwischen den Kupferstiften unten angegeben. Die Porosität der hergestellten elektrischen Verbindungen wurde jeweils mittels der oben angegebenen Messmethode nach der Messung im Stromkreis bestimmt. Die untenstehende Tabelle gibt die Mittelwerte der Porositäten über die 100 Durchführungen für jedes Beispiel und Vergleichsbeispiel an.

### Beispiel 1

Zunächst wurden 86 g eines Metallpulvers der Firma US Research Nanomaterials Inc. bestehend aus Ag-Partikeln mit einer Partikelgröße von 30 bis 50 nm zusammen mit 14 g eines Polymersystem in einem Kneter des Typs Speedmixer der Firma Hauschild & Co KG für 10 Minuten innig vermischt. Das Polymersystem bestand zu 10 Gew.-% aus Butyldiglycol, zu 68,77 Gew.-% aus Diethylsebacat und zu 21,23 Gew.-% aus Vitel 2700B von Bostik Inc. Die erhaltene Paste wurde durch eine 3-Walzenmühle mit Edelstahlwalzen geführt bis eine homogene Paste erhalten wurde.

Die so erhaltene homogene Paste wurde mittels Schablonendruck jeweils auf die Stirnflächen der beiden Kupferstifte aufgetragen. Hierbei wurde eine Nassschichtdicke von 25 µm erreicht. Anschließend wurde die aufgetragene Paste für 15 Minuten bei 80°C getrocknet. Weiter wurden die mit der getrockneten Paste versehenen Kupferstifte so in einer Uniaxialpresse eingebaut, dass die Kupferstifte durch uniaxiales Verpressen über die Paste elektrisch miteinander verbunden werden konnten. Das uniaxiale Verpressen erfolgte bei 250°C für eine Haltedauer von 30 Minuten mit einem Anpressdruck von 20 MPa unter Argon-Schutzgas. Anschließend wurden die Kupferstifte jeweils durch Einführen von abisolierten Kabelenden in Bohrungen in den freien Stirnflächen sowie Fixieren der Kabelenden mittels Schrauben in den Stromkreis eingebracht.

### Beispiel 2

In dem Beispiel 2 wurde wie in Beispiel 1 vorgegangen, wobei die Paste durch Dispensen auf die Stirnflächen der Kupferstifte aufgetragen wurde. Hierbei wurde eine Nassschichtdicke von 35 µm erzielt. Ferner erfolgte das uniaxiale Verpressen für eine Haltezeit von 5 Minuten bei 300°C. Weiter wurde die elektrische Verbindung nach dem Pressen bei 300°C für eine Haltedauer von 30 Minuten unter Argon-Atmosphäre gesintert. Hierzu betrug die Aufheizrate 50 K/min.

### Vergleichsbeispiel 1

In dem Vergleichsbeispiel 1 wurde die elektrische Verbindung zwischen den beiden Kupferstiften durch Verlöten einer Silberscheibe mit Dicke 30 µm und Durchmesser 2,0 mm erzeugt. Das Verlöten läuft unter Vakuum ab, wobei eine Löttemperatur von 980°C für 3 min gehalten wird.

### Vergleichsbeispiel 2

In Vergleichsbeispiel 2 wurde eine Lotpaste Indium 9.72 der Firma Indium Corporation durch Dispensen auf die Kupferstifte aufgetragen und anschließend gemäß den Herstellervorgaben verlötet.

### Auswertung

| | **Beispiel 1** | **Beispiel 2** | **Vergleichs- beispiel 1** | **Vergleichs- beispiel 2** |
|---|---|---|---|---|
| Porosität der elektrischen Verbindung | 0,1 | 0,13 | 0 | 0 |

Zu jedem Beispiel und Vergleichsbeispiel wurden jeweils 100 elektrische Verbindungen wie oben angegeben hergestellt und gemäß dem obigen Verfahren geprüft. Das heißt, es wurde für jedes erfindungsgemäße Beispiel und jedes nichterfindungsgemäße Beispiel eine Ausfallrate bestimmt. Ferner wurde aus den Prüfdurchgängen ohne Ausfall eine mittlere Batterielebensdauer bestimmt. Hieraus ergab sich ein Verhältnis des Mittelwerts der Ausfallraten in den erfindungsgemäßen Beispielen zu einem Mittelwert der Ausfallraten in den nichterfindungsgemäßen Vergleichsbeispielen von 1 : 1,8. Ferner ergab sich für Beispiele und Vergleichsbeispiele ohne Ausfall ein Verhältnis des Mittelwerts der Batterielebensdauern der erfindungsgemäßen Bespiele zu einem Mittelwert der Batterielebensdauern in den nichterfindungsgemäßen Vergleichsbeispielen von 1 : 0,65. Demnach zeigten die erfindungsgemäßen elektrischen Verbindungen eine geringere Ausfallrate und führten zu einer höheren Batterielebensdauer als die nichterfindungsgemäßen Lötverbindungen.

Es zeigen:
- Figur 1a): eine schematische Darstellung einer erfindungsgemäßen Vorrichtung;
- Figur 1b): eine schematische Querschnittsdarstellung der erfindungsgemäßen Vorrichtung in Figur 1a);
- Figur 2: eine schematische Querschnittsdarstellung einer weiteren erfindungsgemäßen Vorrichtung;
- Figur 3: eine schematische Querschnittsdarstellung einer weiteren erfindungsgemäßen Vorrichtung;
- Figur 4: eine schematische Querschnittsdarstellung einer weiteren erfindungsgemäßen Vorrichtung;
- Figur 5: eine schematische Querschnittsdarstellung eines erfindungsgemäßen Vorrichtungsvorläufers;
- Figur 6: ein Ablaufdiagram eines erfindungsgemäßen Verfahrens;
- Figur 7: eine schematische Querschnittsdarstellung eines erfindungsgemäßen implantierbaren elektrischen medizinischen Gerätes;
- Figur 8: eine schematische Querschnittsdarstellung einer nicht erfindungsgemäß kontaktierten Durchführung; und
- Figur 9: eine schematische Darstellung eines Messaufbaus.

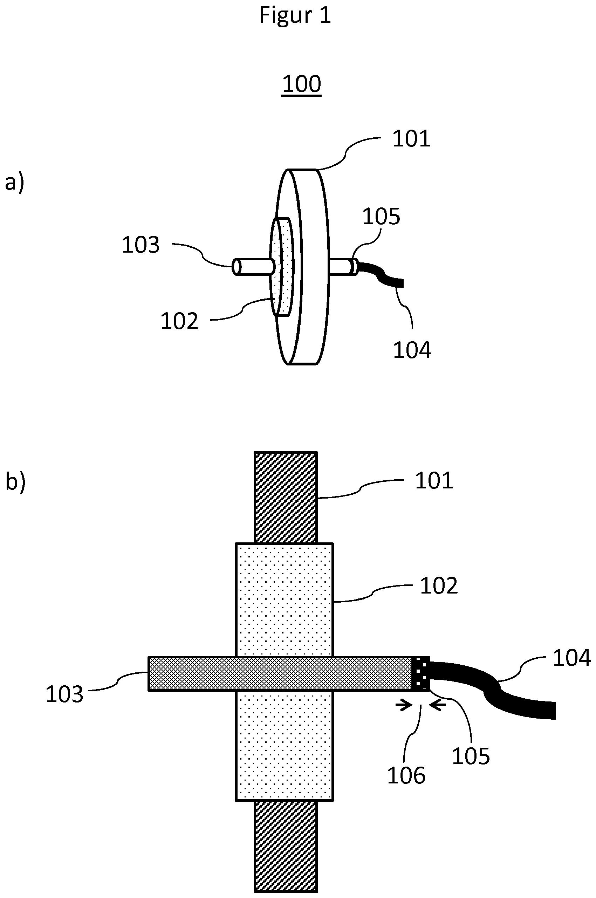

Figur 1a) zeigt eine schematische Darstellung einer erfindungsgemäßen Vorrichtung 100. Die Vorrichtung 100 beinhaltet einen ersten Rahmen 101, einen weiteren Rahmen 102, ein erstes Bauteil 103, ein zweites Bauteil 104 und ein drittes Bauteil 105. Der erste Rahmen 101 ist ein Flansch. Der Flansch besteht aus Titan. Der weitere Rahmen 102 ist eine Durchführungshülse und besteht aus einer elektrisch isolierenden Keramik (Al₂O₃). Die Durchführungshülse ist mit einem Goldlot in den Flansch eingelötet. Das heißt der erste Rahmen 101 umrahmt den weiteren Rahmen 102. In die Durchführungshülse ist wiederum mit einem Goldlot das erste Bauteil 103, ein Anschlussstift, eingelötet. Das heißt der weitere Rahmen 102 umrahmt das erste Bauteil 103. Zudem isoliert die Durchführungshülse den Anschlussstift elektrisch von dem Flansch. Der Anschlussstift besteht aus einer Platin-Iridium-Legierung. Das zweite Bauteil 104, hier ein elektrisch leitendes Kabel, ist elektrisch leitend mit dem dritten Bauteil 105 und dem Anschlussstift verbunden. Diese Verbindung wurde durch einen Sintervorgang, in dem das dritte Bauteil 105 erhalten wurde, hergestellt. Das dritte Bauteil 105 hat eine Porosität von 0,1. Das dritte Bauteil 105 besteht zu 99 Gew.-% bezogen auf das dritte Bauteil 105 aus Platin.

Figur 1b) zeigt eine schematische Querschnittsdarstellung der erfindungsgemäßen Vorrichtung 100 in Figur 1a). Zusätzlich zu Figur 1a) ist in Figur 1b) eine Schichtdicke 106 des dritten Bauteils 105 dargestellt. Das heißt das dritte Bauteil 105 bildet eine Schicht, welche den Anschlussstift überlagert.

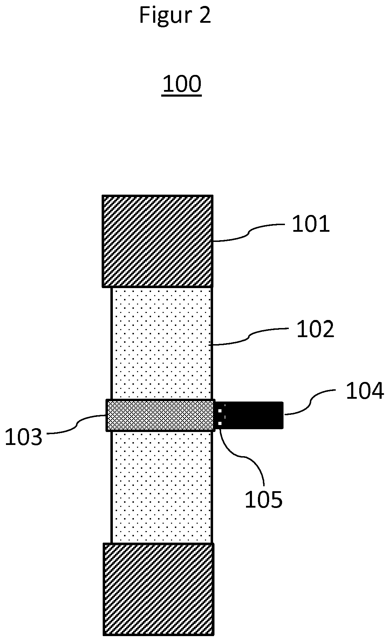

Figur 2 zeigt eine schematische Querschnittsdarstellung einer weiteren erfindungsgemäßen Vorrichtung 100. Die Vorrichtung 100 beinhaltet einen ersten Rahmen 101, einen weiteren Rahmen 102, ein erstes Bauteil 103, ein zweites Bauteil 104 und ein drittes Bauteil 105. Der erste Rahmen 101 ist ein Flansch. Der Flansch besteht aus Titan. Der weitere Rahmen 102 ist ein Ring aus einer Keramik (Al₂O₃). Der weitere Rahmen 102 ist in den Flansch mit einem Goldlot eingelötet. Eine Ringöffnung des Rings wird von dem ersten Bauteil 103, einem Anschlussstift, durchstoßen. Das erste Bauteil 103 besteht aus einem elektrisch leitenden Cermet. Das erste Bauteil 103 und der weitere Rahmen 102 sind einstückig in einem Sinterprozess erhalten worden. Das zweite Bauteil 104 ist ein Pin zum elektrischen Kontaktieren einer Leiterplatte. Das dritte Bauteil 105 verbindet den Pin und den Anschlussstift elektrisch leitend. Diese Verbindung wurde durch einen Sintervorgang, in dem das dritte Bauteil 105 erhalten wurde, hergestellt. Das dritte Bauteil 105 hat eine Porosität von 0,1. Die elektrische Verbindung hat einen Widerstand von 0,1 mΩ. Ferner beinhaltet das dritte Bauteil 105 keinen Meniskus. Das dritte Bauteil 105 besteht zu 99,5 Gew.-% bezogen auf das dritte Bauteil 105 aus Silber.

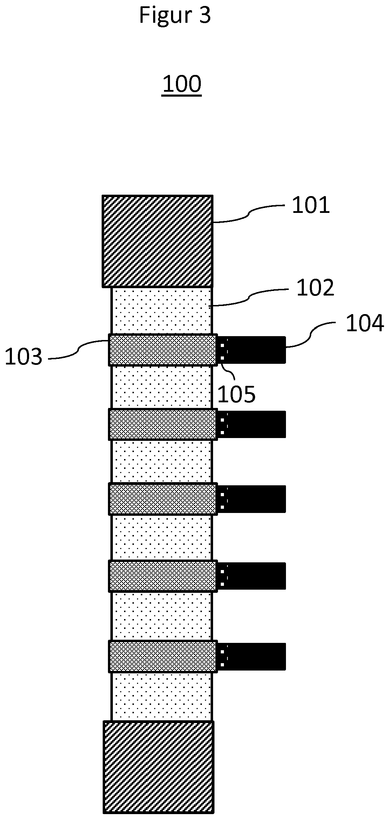

Figur 3 zeigt eine schematische Querschnittsdarstellung einer weiteren erfindungsgemäßen Vorrichtung 100. Die Vorrichtung 100 beinhaltet einen ersten Rahmen 101, einen weiteren Rahmen 102, ein erstes Bauteil 103, ein zweites Bauteil 104 und ein drittes Bauteil 105. Der erste Rahmen 101 ist ein Flansch. Der Flansch besteht aus Titan. Der weitere Rahmen 102 ist eine Lochplatte aus einer Keramik (Al₂O₃). Der weitere Rahmen 102 ist in den Flansch mit einem Goldlot eingelötet. Ein Loch der Lochplatte wird von dem ersten Bauteil 103, einem Anschlussstift, durchstoßen. Das erste Bauteil 103 besteht aus einem elektrisch leitenden Cermet. Das erste Bauteil 103 und der weitere Rahmen 102 sind einstückig in einem Sinterprozess erhalten worden. Das zweite Bauteil 104 ist ein Pin zum elektrischen Kontaktieren einer Leiterplatte. Das dritte Bauteil 105 verbindet den Pin und den Anschlussstift elektrisch leitend. Diese Verbindung wurde durch einen Sintervorgang, in dem das dritte Bauteil 105 erhalten wurde, hergestellt. Das dritte Bauteil 105 hat eine Porosität von 0,1. Die elektrische Verbindung hat einen Widerstand von 0,1 mΩ. Ferner beinhaltet das dritte Bauteil 105 keinen Meniskus. Das dritte Bauteil 105 besteht zu 99,5 Gew.-% bezogen auf das dritte Bauteil 105 aus Silber. Weiter beinhaltet die Vorrichtung 100 eine Pluralität weiterer Anschlussstifte, weiterer dritter Bauteile 105 und weiterer Pins. Jedes weitere dritte Bauteil 105 verbindet einen weiteren Anschlussstift elektrisch mit einem weiteren Pin. Jedes weitere dritte Bauteil hat eine Porosität in einem Bereich von 0,05 bis 0,15.

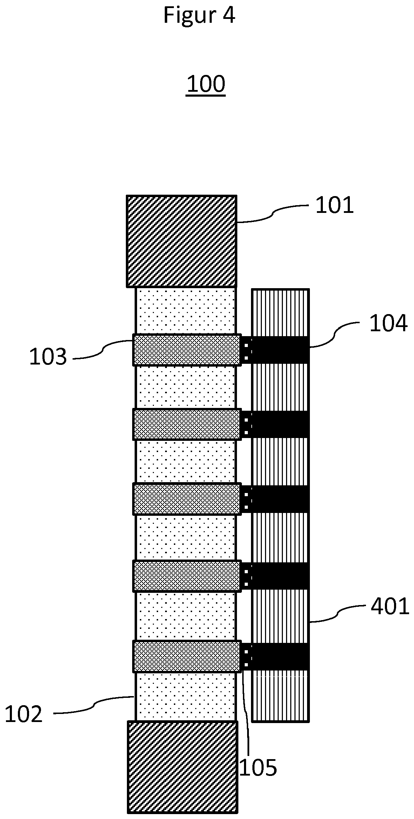

Figur 4 zeigt eine schematische Querschnittsdarstellung einer weiteren erfindungsgemäßen Vorrichtung 100. Diese Vorrichtung 100 ist die Vorrichtung in Figur 3, wobei die Pins jeweils mit einem MLCC-Filter 401 verbunden sind. Die MLCC-Filter 401 vermindern ein Übertragen von elektrischen Störsignalen von den Anschlussstiften und den Pins an eine elektronische Schaltung, die an die Pins angeschlossen ist.

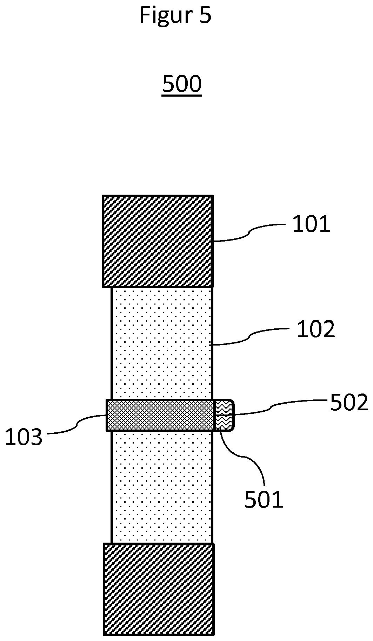

Figur 5 zeigt eine schematische Querschnittsdarstellung eines erfindungsgemäßen Vorrichtungsvorläufers 500. Der Vorrichtungsvorläufer 500 beinhaltet einen ersten Rahmen 101, einen weiteren Rahmen 102, und ein erstes Bauteil 103. Der erste Rahmen 101 ist ein Flansch. Der Flansch besteht aus Titan. Der weitere Rahmen 102 ist ein Ring aus einer Keramik (Al₂O₃). Der weitere Rahmen 102 ist in den Flansch mit einem Goldlot eingelötet. Eine Ringöffnung des Rings wird von dem ersten Bauteil 103, einem Anschlussstift, durchstoßen. Das erste Bauteil 103 besteht aus einem elektrisch leitenden Cermet. Das erste Bauteil 103 und der weitere Rahmen 102 sind einstückig in einem Sinterprozess erhalten worden. Der Anschlussstift beinhaltet eine erste Kontaktoberfläche 502. Auf diese erste Kontaktoberfläche 502 ist eine Zusammensetzung 501 aufgebracht. Die Zusammensetzung 501 ist eine Paste, beinhaltend ein Bindemittel, ein Lösungsmittel und eine Vielzahl von Metallpartikeln. Die Metallpartikel sind Goldpartikel. Die Paste beinhaltet die Goldpartikel zu einem Anteil von 90 Gew.-% bezogen auf das Gewicht der Paste.

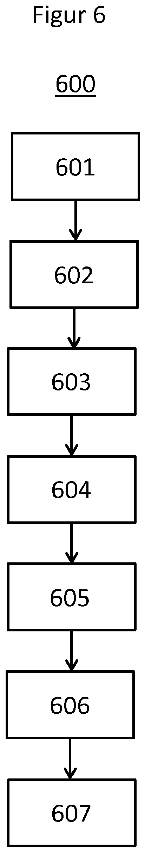

Figur 6 zeigt ein Ablaufdiagram eines erfindungsgemäßen Verfahrens 600. Das Verfahren 600 beinhaltet Verfahrensschritte a) 601 bis g) 607. In Verfahrensschritt a) 601 wird eine Durchführung bereitgestellt. Die Durchführung beinhaltet einen ersten Rahmen 101, einen weiteren Rahmen 102, und ein erstes Bauteil 103. Der erste Rahmen 101 ist ein Flansch. Der Flansch besteht aus Titan. Der weitere Rahmen 102 ist ein Ring aus einer Keramik (Al₂O₃). Der weitere Rahmen 102 ist in den Flansch mit einem Goldlot eingelötet. Eine Ringöffnung des Rings wird von dem ersten Bauteil 103, einem Anschlussstift, durchstoßen. Das erste Bauteil 103 besteht aus einem elektrisch leitenden Cermet. Das erste Bauteil 103 und der weitere Rahmen 102 sind einstückig in einem Sinterprozess erhalten worden. In dem Verfahrensschritt b) 602 wird ein zweites Bauteil 104, beinhaltend eine erste Kontaktoberfläche bereitgestellt. Das zweite Bauteil 102 ist ein elektrisches Kabel, beinhaltend eine weitere Kontaktoberfläche. Die weitere Kontaktoberfläche ist eine Stirnseite eines Kabelendes. In dem Verfahrensschritt c) 603 wird eine Zusammensetzung 501 bereitgestellt. Die Zusammensetzung 501 ist eine Paste, beinhaltend ein Bindemittel und eine Vielzahl von Metallpartikeln. Die Metallpartikel sind Silberpartikel. Die Paste beinhaltet die Silberpartikel zu einem Anteil von 90 Gew.-% bezogen auf das Gewicht der Paste. In dem Verfahrensschritt d) 604 wird die Paste in einem Schablonendruckverfahren auf die erste Kontaktoberfläche 502 aufgedruckt. In dem Verfahrensschritt e) 605 wird das Kabel über seine weitere Kontaktoberfläche mit der Paste kontaktiert und somit die erste Kontaktoberfläche 502 mit der weiteren Kontaktoberfläche verbunden. In dem Verfahrensschritt f) 606 wird die Paste thermisch entbindert. Dazu wird die Paste für 10 Minuten auf 100°C erwärmt. Es wird ein Bauteilvorläufer, ein Grünling, erhalten. In dem Verfahrensschritt g) 607 wird der Grünling gesintert. Dazu wird der Grünling für 2 Minuten auf eine Temperatur von 300°C erhitzt. Dabei wird auf den Grünling ein mechanischer Druck von 20 MPa ausgeübt. Während des Sinterns befindet sich der Grünling in Luft. Es wird durch das Sintern ein erfindungsgemäßes drittes Bauteil 105 erhalten.

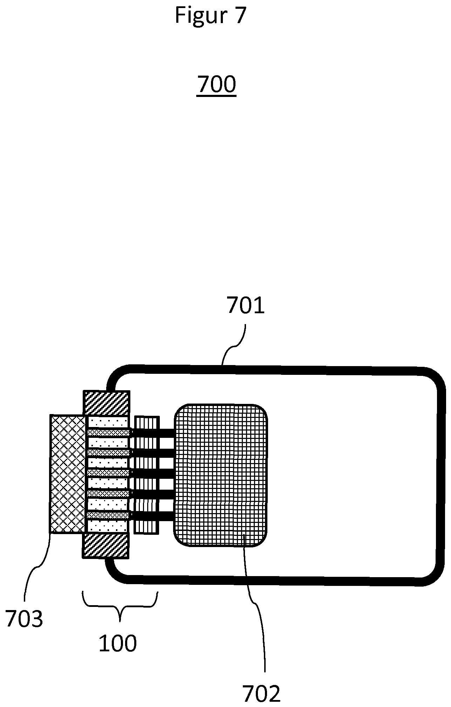

Figur 7 zeigt eine schematische Querschnittsdarstellung eines erfindungsgemäßen implantierbaren elektrischen medizinischen Gerätes 700. Das implantierbare elektrische medizinische Gerät 700 ist ein Herzschrittmacher. Der Herzschrittmacher beinhaltet ein Gehäuse 701 aus Titan, darin untergebracht eine Steuerelektronik 702, sowie in eine Gehäuseöffnung hermetisch dicht eingeschweißt die erfindungsgemäße Vorrichtung 100 aus Figur 4. Hierbei verbinden die Pins die Anschlussstifte und die Steuerelektronik 702 elektrisch miteinander. An eine Außenseite der Anschlussstifte ist ein Headerblock 703 angeschlossen.

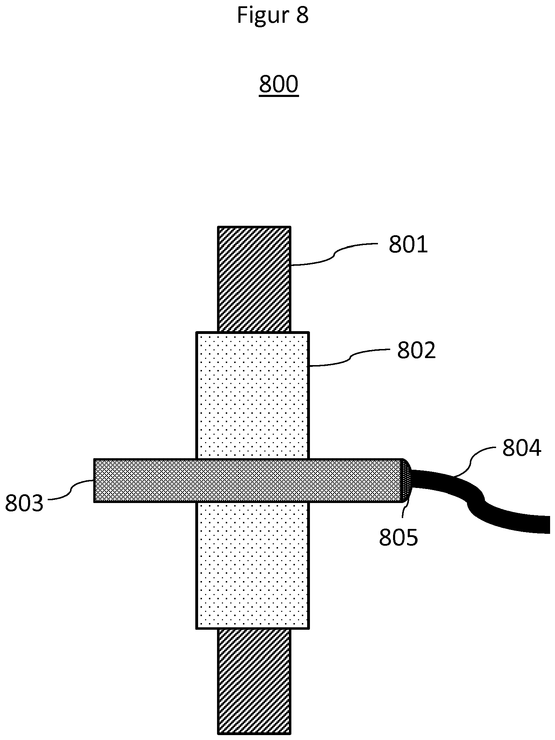

Figur 8 zeigt eine schematische Querschnittsdarstellung einer nicht erfindungsgemäß kontaktierten Durchführung 800. Die Durchführung 800 beinhaltet eine Flansch 801, darin eingelötet eine Durchführungshülse 802, darin eingelötet einen Anschlussstift 803. Der Anschlussstift 803 ist an einem Ende über eine Lötstelle 805 an ein Kabel 804 angelötet. Die Lötstelle 805 beinhaltet einen Meniskus 805, welcher sich durch eine Grenzflächenspannung eines flüssigen Lots beim Löten gebildet hat und bei einem Erstarren des Lots Teil der festen Lötstelle 805 wurde.

Figur 9 zeigt eine schematische Darstellung eines Messaufbaus 900 zur Messung der Ausfallrate der elektrischen Verbindungen sowie der Batterielebensdauer in den Beispielen. Gezeigt ist ein Stromkreis, beinhaltend eine Spannungsquelle 901, einen Verbraucher 902 und einen Schalter 903. Ferner beinhaltet der Stromkreis 2 Kupferstifte 904 mit jeweils einer Bohrung in Längsrichtung des Kupferstifts 904. In jede Bohrung ist ein abisoliertes Kabelende des Stromkreises eingeführt und mittels Schraube fixiert. Ferner sind die freien Stirnflächen der Kuperstifte 904 mittels der zu prüfenden elektrischen Verbindung miteinander verbunden. Dies ist in den erfindungsgemäßen Beispielen eine Sinterverbindung gemäß dem erfindungsgemäßen dritten Bauteil 105. Ferner sind für eine Messung der Ausfallrate die beiden Kupferstifte 904 sowie mit der elektrischen Verbindung auf einem Rütteltisch 905 fixiert.

### LISTE DER BEZUGSZEICHEN

- **100**: erfindungsgemäße Vorrichtung
- **101**: erster Rahmen
- **102**: weiterer Rahmen
- **103**: erstes Bauteil
- **104**: zweites Bauteil
- **105**: drittes Bauteil
- **106**: Schichtdicke
- **401**: elektrotechnischer Filter
- **500**: erfindungsgemäßer Vorrichtungsvorläufer
- **501**: Zusammensetzung
- **502**: erste Kontaktoberfläche
- **600**: erfindungsgemäßes Verfahren
- **601**: Verfahrensschritt a)
- **602**: Verfahrensschritt b)
- **603**: Verfahrensschritt c)
- **604**: Verfahrensschritt d)
- **605**: Verfahrensschritt e)
- **606**: Verfahrensschritt f)
- **607**: Verfahrensschritt g)
- **700**: erfindungsgemäßes implantierbares elektrisches medizinisches Gerät
- **701**: Gehäuse
- **702**: Steuerelektronik
- **703**: Headerblock
- **800**: nichterfindungsgemäß kontaktierte Durchführung
- **801**: Flansch
- **802**: Durchführungshülse
- **803**: Anschlussstift
- **804**: Kabel
- **805**: Lötstelle mit Meniskus
- **900**: Messaufbau
- **901**: Spannungsquelle
- **902**: Verbraucher
- **903**: Schalter
- **904**: Kupferstift
- **905**: Rütteltisch

## Patentansprüche

1. Eine Vorrichtung (100), beinhaltend einen ersten Rahmen (101), einen weiteren Rahmen (102), ein erstes Bauteil (103), ein zweites Bauteil (104) und ein drittes Bauteil (105);
wobei
a) der erste Rahmen (101) den weiteren Rahmen (102) umrahmt;
b) der weitere Rahmen (102)
i) das erste Bauteil (103) umrahmt, und
ii) das erste Bauteil (103) und den ersten Rahmen (101) elektrisch voneinander isoliert;
c) das erste Bauteil (103) elektrisch leitend ist;
d) das zweite Bauteil (104) elektrisch leitend ist; und
e) das dritte Bauteil (105) eine elektrisch leitende Verbindung zwischen dem ersten Bauteil (103) und dem zweiten Bauteil (104) herstellt;
**dadurch gekennzeichnet, dass** das dritte Bauteil (105) eine Porosität in einem Bereich von 0,001 bis 0,4 hat.

2. Die Vorrichtung (100) nach Anspruch 1, wobei das erste Bauteil (103) ein Cermet beinhaltet.

3. Die Vorrichtung (100) nach Anspruch 1 oder 2, wobei der weitere Rahmen (102) eine Keramik beinhaltet.

4. Die Vorrichtung (100) nach einem der vorhergehenden Ansprüche, wobei der weitere Rahmen (102) und das erste Bauteil (103) einstückig ausgebildet sind.

5. Die Vorrichtung (100) nach einem der vorhergehenden Ansprüche, wobei das zweite Bauteil (104) eines ausgewählt aus der Gruppe bestehend aus einem elektrischen Kontakt, einer Leiterbahn, einem Draht, einer Buchse, und einem Stecker, oder eine Kombination von mindestens zwei davon ist.

6. Die Vorrichtung (100) nach einem der vorhergehenden Ansprüche, wobei das erste Bauteil (103) oder das zweite Bauteil (104) oder beide mit einem elektrotechnischen Filter (401) verbunden ist.

7. Ein Vorrichtungsvorläufer (500), beinhaltend einen ersten Rahmen (101), einen weiteren Rahmen (102) und ein erstes Bauteil (103);
wobei
a) der erste Rahmen (101) den weiteren Rahmen (102) umrahmt;
b) der weitere Rahmen (102)
i) das erste Bauteil (103) umrahmt, und
ii) das erste Bauteil (103) und den ersten Rahmen (101) elektrisch voneinander isoliert; und
c) das erste Bauteil (103)
i) elektrisch leitend ist, und
ii) eine erste Kontaktoberfläche (502) beinhaltet;
wobei die erste Kontaktoberfläche (502) mindestens teilweise mit einer Zusammensetzung (501), beinhaltend eine Flüssigkeit und eine Vielzahl von Metallpartikeln, überlagert ist.

8. Ein Verfahren (600), beinhaltend als Verfahrensschritte (601 bis 607):
a) Bereitstellen einer Durchführung, beinhaltend einen ersten Rahmen (101), einen weiteren Rahmen (102), und ein erstes Bauteil (103),
wobei
i) der erste Rahmen (101) den weiteren Rahmen (102) umrahmt,
ii) der weitere Rahmen (102)
A. das erste Bauteil (103) umrahmt, und
B. den ersten Rahmen (101) und das erste Bauteil (103) elektrisch voneinander isoliert, und
iii) das erste Bauteil (103)
A. elektrisch leitend ist, und
B. eine erste Kontaktoberfläche (502) beinhaltet;
b) Bereitstellen eines zweiten Bauteils (104), beinhaltend eine weitere Kontaktoberfläche;
c) Bereitstellen einer Zusammensetzung (501), beinhaltend eine Flüssigkeit und eine Vielzahl von Metallpartikeln,
wobei die Metallpartikel ein Metall mit einer niedrigsten Schmelztemperatur beinhalten;
d) Überlagern der ersten Kontaktoberfläche (502) oder der weiteren Kontaktoberfläche oder beider mit der Zusammensetzung (501);
e) Verbinden der ersten Kontaktoberfläche (502) und der weiteren Kontaktoberfläche über die Zusammensetzung (501);
f) Verringern des Anteils der Flüssigkeit an der Zusammensetzung (501) unter Erhalt eines Bauteilvorläufers; und
g) Erwärmen des Bauteilvorläufers auf eine Temperatur in einem Bereich von 10 bis 90 % der niedrigsten Schmelztemperatur unter Erhalt eines dritten Bauteils (105).

9. Das Verfahren (600) nach Anspruch 8, wobei das Verringern in Verfahrensschritt f) ein Erwärmen der Zusammensetzung (501) auf eine Temperatur in einem Bereich von 50 bis 160 °C beinhaltet.

10. Das Verfahren (600) nach Anspruch 8 oder 9, wobei Verfahrensschritt g) eines oder mindestens zwei der folgenden Kriterien erfüllt:
a) der Bauteilvorläufer wird in Verfahrensschritt g) einem mechanischen Druck in einem Bereich von 0 bis 50 MPa ausgesetzt;
b) während das dritte Bauteil (105) erhalten wird befindet sich der Bauteilvorläufer oder das dritte Bauteil (105) oder beide in einer Schutzgasatmosphäre;
c) ein Abstand der ersten Kontaktoberfläche (502) von der weiteren Kontaktoberfläche verringert sich in Verfahrensschritt g) um 0,01 bis 5 %, bezogen auf den Abstand unmittelbar vor dem Verfahrensschritt g), auf einen Wert in einem Bereich von 0,005 bis 1 mm;
d) die in Verfahrensschritt g) genannte Temperatur wird für eine Zeitdauer in einem Bereich von 1 bis 120 min gehalten.

11. Ein implantierbares elektrisches medizinisches Gerät (700), beinhaltend ein Gehäuse (701) und eine Vorrichtung (100) nach einem der Ansprüche 1 bis 6;
wobei das Gehäuse (701) eine Gehäuseöffnung beinhaltet;
wobei die Gehäuseöffnung den ersten Rahmen (101) beinhaltet.

12. Ein Verfahren zum Herstellen eines implantierbaren elektrischen medizinischen Geräts, beinhaltend ein Gehäuse (701), beinhaltend eine Gehäuseöffnung, wobei in einem Verfahrensschritt eine Vorrichtung (100) nach einem der Ansprüche 1 bis 6, oder ein Vorrichtungsvorläufer (500) nach Anspruch 7 so in die Gehäuseöffnung eingebracht wird, dass die Gehäuseöffnung den ersten Rahmen (101) beinhaltet.

13. Eine Verwendung einer Zusammensatzung (501), beinhaltend eine Flüssigkeit und eine Vielzahl von Metallpartikeln, zu einem elektrisch leitenden Verbinden eines Durchführungselements und eines elektrischen Leiters;
wobei die Verwendung ein Sintern der Zusammensetzung (501) beinhaltet.

## Claims

1. An apparatus (100) comprising a first frame (101), a further frame (102), a first element (103), a second element (104) and a third element (105);
wherein
a) the first frame (101) frames the further frame (102);
b) the further frame (102)
i) frames the first element (103), and
ii) electrically insulated the first element (103) and the first frame (101) from each other;
c) the first element (103) is electrically conductive;
d) the second element (104) is electrically conductive; and
e) the third element (105) provides an electrically conductive connection between the first element (103) and the second element (104);
**characterized in that** said third element (105) has a porosity in a range of 0.001 to 0.4.

2. The apparatus (100) according to claim 1, wherein the first element (103) comprises a cermet.

3. The apparatus (100) according to claim 1 or 2, the further frame (102) comprising a ceramic.

4. The apparatus (100) according to one of the preceding claims, wherein the further frame (102) and the first element (103) are configured in one piece.

5. The apparatus (100) according to one of the preceding claims, wherein the second element (104) is selected from the group consisting of an electrical contact, a conductive path, a wire, a socket, and a plug, or a combination of at least two thereof.

6. The apparatus (100) according to one of the preceding claims, wherein the first element (103) or the second element (104) or both is connected to an electrotechnical filter (401).

7. An apparatus precursor (500) comprising a first frame (101), a further frame (102) and a first element (103);
wherein
a) the first frame (101) frames the further frame (102);
b) the further frame (102)
i) frames the first element (103), and
ii) electrically insulates the first element (103) and the first frame (101) from each other; and
c) the first element (103)
i) is electrically conductive, and
ii) comprises a first contact surface (502);
wherein the first contact surface (502) is at least partially superimposed with a composition (501) comprising a liquid and a plurality of metal particles.

8. A process (600) comprising as process steps (601 to 607):
a) provision a feedthrough comprising a first frame (101), a further frame (102), and a first element (103),
wherein
i) the first frame (101) frames the further frame (102),
ii) the further frame (102)
A. frames the first element (103), and
B. electrically insulates said first frame (101) and said first element (103) from each other, and
iii) the first element (103)
A. is electrically conductive, and
B. comprises a first contact surface (502);
b) provision a second element (104) comprising a further contact surface;
c) provision a composition (501) comprising a liquid and a plurality of metal particles, said metal particles comprising a metal having a lowest melting temperature;
d) superimposing the first contact surface (502) or the further contact surface or both with the composition (501);
e) connecting the first contact surface (502) and the further contact surface via the composition (501);
f) reducing the proportion of liquid in the composition (501) thereby obtaining an element precursor; and
g) Heating the element precursor to a temperature in the range of 10 to 90% of the lowest melting temperature thereby obtaining a third element (105).

9. The process (600) according to claim 8, wherein the reducing in process step f) comprises heating the composition (501) to a temperature in a range of 50 to 160°C.

10. The process (600) according to claim 8 or 9, where step (g) meets one or at least two of the following criteria:
a) the element precursor is subjected to a mechanical pressure in the range from 0 to 50 MPa in process step g);
b) while the third element (105) is obtained, the element precursor or the third element (105) or both are in a protective gas atmosphere;
c) a distance of the first contact surface (502) from the further contact surface is reduced in process step g) by 0.01 to 5%, based on the distance immediately prior to process step g), to a value in a range from 0.005 to 1 mm;
d) the temperature specified in process step g) is maintained in a range from 1 to 120 min for a period of time.

11. An implantable electrical medical apparatus (700) comprising a housing (701) and an apparatus (100) according to any one of claims 1 to 6;
wherein the housing (701) comprises a housing opening;
the housing opening comprising the first frame (101).

12. A method of manufacturing an implantable electrical medical apparatus comprising a housing (701) comprising a housing opening, wherein in a method step an apparatus (100) according to one of claims 1 to 6, or an apparatus precursor (500) according to claim 7, is inserted into the housing opening such that the housing opening comprises the first frame (101).

13. Use of a composition (501) comprising a liquid and a plurality of metal particles for electrically connecting a feedthrough element and an electrical conductor;
Wherein the use comprises a sintering of the composition (501).

## Revendications

1. Dispositif (100) comprenant un premier cadre (101), un autre cadre (102), un premier élément (103), un deuxième élément (104) et un troisième élément (105) ;
dans lequel
a) le premier cadre (101) encadre l'autre cadre (102) ;
b) l'autre cadre (102)
i) encadre le premier élément (103), et
ii) le premier élément (103) et le premier cadre (101) sont électriquement isolés l'un de l'autre ;
c) le premier élément (103) est électriquement conducteur ;
d) le second élément (104) est électriquement conducteur ; et
e) le troisième élément (105) établit une connexion électriquement conductrice entre le premier élément (103) et le deuxième élément (104) ;
**caractérisé en ce que** ledit troisième élément (105) a une porosité dans une plage de 0,001 à 0,4.

2. Dispositif (100) selon la revendication 1, dans lequel le premier élément (103) comprend un cermet.

3. Dispositif (100) selon la revendication 1 ou 2, le cadre supplémentaire (102) comprenant une céramique.

4. Dispositif (100) selon l'une des revendications précédentes, dans lequel le cadre supplémentaire (102) et le premier élément (103) sont formés intégralement.

5. Dispositif (100) selon l'une des revendications précédentes, dans lequel le second élément (104) est un élément choisi dans le groupe constitué par un contact électrique, une piste conductrice, un fil, une douille et une fiche, ou une combinaison d'au moins deux de ceux-ci.

6. Dispositif (100) selon l'une des revendications précédentes, dans lequel le premier élément (103) ou le second élément (104) ou les deux sont reliés à un filtre électrotechnique (401).

7. Précurseur de dispositif (500) comprenant un premier cadre (101), un autre cadre (102) et un premier élément (103) ;
dans lequel
a) le premier cadre (101) encadre l'autre cadre (102) ;
b) l'autre cadre (102)
i) encadre le premier élément (103), et
ii) le premier élément (103) et le premier cadre (101) sont électriquement isolés l'un de l'autre ; et
c) le premier élément (103)
i) est électriquement conducteur, et
ii) comprend une première surface de contact (502) ;
dans laquelle la première surface de contact (502) est au moins partiellement recouverte d'une composition (501) comprenant un liquide et une pluralité de particules métalliques.

8. Procédé (600) comprenant comme étapes de procédé (601 à 607) :
a) fournir une traversée comprenant un premier cadre (101), un autre cadre (102) et un premier élément (103),
dans lequel
i) le premier cadre (101) encadre l'autre cadre (102),
ii) l'autre cadre (102)
A. encadre le premier élément (103), et
B. isole électriquement ledit premier cadre (101) et ledit premier élément (103) l'un de l'autre, et
iii) le premier élément (103)
A. est électriquement conducteur, et
B. comprend une première surface de contact (502) ;
b) Fourniture d'un deuxième élément (104) comprenant une surface de contact supplémentaire ;
c) fournir une composition (501) comprenant un liquide et une pluralité de particules métalliques,
lesdites particules métalliques comprenant un métal ayant une température de fusion la plus basse ;
d) superposer la première surface de contact (502) ou l'autre surface de contact ou les deux avec la composition (501) ;
e) relier la première surface de contact (502) et l'autre surface de contact par l'intermédiaire de la composition (501) ;
f) réduire la proportion de liquide dans la composition (501) tout en conservant un précurseur de élément ; et
g) Chauffage du précurseur d'élément à une température comprise entre 10 et 90 % de la température de fusion la plus basse pour obtenir un troisième élément (105).

9. Procédé (600) selon la revendication 8, dans lequel la réduction dans l'étape de procédé f) comprend le chauffage de la composition (501) à une température comprise entre 50 et 160°C.

10. Procédé (600) selon la revendication 8 ou 9, dans lequel l'étape (g) remplit un ou au moins deux des critères suivants :
a) le précurseur de élément est soumis à une pression mécanique dans la plage de 0 à 50 MPa dans l'étape de procédé g) ;
b) pendant que le troisième élément (105) est obtenu, le précurseur du élément ou le troisième élément (105) ou les deux se trouvent dans une atmosphère de gaz protecteur ;
c) une distance de la première surface de contact (502) par rapport à l'autre surface de contact est réduite dans l'étape de procédé g) de 0,01 à 5 %, sur la base de la distance immédiatement avant l'étape de procédé g), à une valeur comprise entre 0,005 et 1 mm ;
d) la température spécifiée à l'étape g) du procédé est maintenue dans une plage de 1 à 120 min pendant un certain temps.

11. Dispositif médical électrique implantable (700) comprenant un boîtier (701) et un dispositif (100) selon l'une quelconque des revendications 1 à 6 ;
dans laquelle le boîtier (701) comprend une ouverture de boîtier ;
l'ouverture du boîtier, y compris le premier cadre (101).

12. Procédé de fabrication d'un dispositif médical électrique implantable comprenant un boîtier (701) comprenant une ouverture de boîtier, dans lequel, dans une étape de procédé, un dispositif (100) selon l'une des revendications 1 à 6, ou un précurseur de dispositif (500) selon la revendication 7, est inséré dans l'ouverture du boîtier de sorte que l'ouverture du boîtier comprend le premier châssis (101).

13. Utilisation d'une composition (501) comprenant un liquide et une pluralité de particules métalliques pour connecter électriquement un élément traversant et un conducteur électrique ;
dans laquelle l'utilisation comprend le frittage de la composition (501).
